(19) **Europäisches Patentamt**
**European Patent Office**
**Office européen des brevets**

(11) **EP 4 332 240 A1**

(12) **EUROPEAN PATENT APPLICATION**

(43) Date of publication:
**06.03.2024 Bulletin 2024/10**

(21) Application number: **22193923.4**

(22) Date of filing: **05.09.2022**

(51) International Patent Classification (IPC):
***C12Q 1/6886*** (2018.01)

(52) Cooperative Patent Classification (CPC):
**C12Q 1/6886;** C12Q 2600/118; C12Q 2600/156;
C12Q 2600/158

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
 • **Fraunhofer-Gesellschaft zur Förderung
  der angewandten Forschung e.V.
  80686 München (DE)**
 • **Universität Leipzig
  04109 Leipzig (DE)**

(72) Inventors:
 • **Reiche, Kirstin
  04416 Markleeberg (DE)**
 • **Buschmann, Tilo
  04103 Leipzig (DE)**
 • **Bartsch, Sophie
  04107 Leipzig (DE)**
 • **Bertram, Catharina
  39319 Jerichow (DE)**

 • **Horn, Friedemann
  04316 Leipzig (DE)**
 • **Blumert, Conny
  04109 Leipzig (DE)**
 • **Wirth, Manfred
  01326 Dresden (DE)**
 • **Füssel, Susanne
  01309 Dresden (DE)**
 • **Fröhner, Michael
  01307 Dresden (DE)**
 • **Kreuz, Markus
  04229 Leipzig (DE)**
 • **Dominik, Otto
  09648 Mittweida (DE)**

(74) Representative: **CH Kilger Anwaltspartnerschaft
  mbB
  Fasanenstraße 29
  10719 Berlin (DE)**

Remarks:
The complete document including Reference
Table(s) and the Sequence Listing(s) can be
downloaded from the EPO website

(54) **RNA-BIOMARKERS FOR DIAGNOSIS OF PROSTATE CANCER**

(57) The present invention involves methods for the ex-vivo diagnosis of prostate cancer comprising the steps of i) providing a sample from a patient suspected of having prostate cancer and ii) analysing the expression level of at least one newly identified biomarker for prostate cancer in the sample, wherein, if the expression level of said biomarker is above a threshold value, the sample is designated as prostate cancer positive. Further, the invention involves stringent sample and method quality control criteria to ensure a reliable and specific method of diagnosing prostate cancer.

**EP 4 332 240 A1**

**(Cont. next page)**

**Figure 1**

# ProstaCheck
# cohorts

Discovery – RNA Seq
RP-cryopreserved tissue (n = 48)
BPH = 8, Tu = 40

⇩

Confirmation – Microarrays
RP-cryopreserved tissue (n = 203)
BPH = 39, Tu = 164

⇩

Confirmation in Urine – RNA Seq
Urinary Sediment (n = 11)
BPH = 6, Tu = 5

⇩

Training 1 – RT qPCR
Urinary sediment (n = 66)
BPH = 21, Tu = 45

⇩

Training 2 – RT qPCR
Urinary sediment (n = 120)
BPH = 38, Tu = 82

⇩

Training 3 – RT qPCR
Urinary sediment (n = 65)
BPH = 24, Tu = 41

⇩

Test – RT qPCR (IVC)
Urinary sediment (n = 87)
BPH = 43, Tu = 44

**Description**

**Field of the invention**

The present invention is in the field of biology and chemistry. In particular, the invention is in the field of molecular biology. More particularly, the invention relates to the analysis of RNA transcripts. Most particularly, the invention is in the field of diagnosing prostate cancer.

**Background of the invention**

Prostate cancer (PCa) is the most common malignant disease in men and third leading cause of cancer-related deaths in the Western world. In 2020, the annual number of newly diagnosed PCa cases was second leading in Western Europe. Despite widespread screening for prostate cancer and major advances in the treatment of metastatic disease, PCa remains in 2020 the third most common cause of cancer death for men in Europe.

Currently, testing of prostate-specific antigen (PSA) serum levels and the digital rectal examination represent the two major screening methods. Patients showing abnormal results usually are advised to have a prostate biopsy performed. This has however significant consequences. The lack of specificity of PSA screening which produces high numbers of false positives causes unnecessary prostate biopsies performed annually on millions of men worldwide (overdiagnosis). In addition, taking biopsies carries a substantial risk for infectious complications. Therefore, there is an urgent need for a more sensitive and specific diagnostic assay for early prostate cancer diagnosis to improve prostate cancer screening and to avoid the high numbers of unnecessarily taken prostate biopsies. The present invention addresses this problem by providing a set of biomarkers for the screening and diagnosis of prostate cancer.

Prostate-specific antigen

Currently, the most common screening assay for PCa is the measurement of blood serum concentrations of PSA. However, there is no single threshold value for PSA that can reliably distinguish patients with PCa from those without. The lack of specificity of PSA testing is illustrated by a high false-positive rate of up to 75% (Duffy, 2020). The areas under the ROC curve (AUC) value for the PSA test to discriminate PCa from no cancer was reported as 0.678 (Thompson, 2005). Although there has been significant research into improving the performance of PSA itself, including measuring free PSA or truncated forms of PSA (Jansen, 2009), all the problems are unresolvable. Based on this evidence, the US Preventative Services Task Force has recommended against PSA-based prostate cancer screening on the basis of high false-positive rates and the risks associated with biopsies and over-treatment (Moyer, 2012).

PSA-derived biomarkers include the Prostate Health Index (PHI, Jansen, 2010) and the four Kallikrein panel (4K Score, Vickers, 2008). Both combine information of serum levels of different PSA protein variants, the latter one additionally measures kallikrein 2. Related studies primarily focus on high-grade PCa (GS $\geq$ 7) to predict aggressive tumours (Duffy, 2020).

Prostate cancer antigen 3

Prostate cancer antigen 3 (PCA3), a noncoding RNA with expression confined to the prostate, is overexpressed in 95% of PCa samples compared with normal or benign hyperplastic prostate tissue (Salagierski, 2012). The Progensa PCA3 assay (Gen Probe Inc., San Diego, CA, USA) is a commercially available diagnostic test that quantitatively detects PCA3 in urine and prostatic fluid. A PCA3 score >35 in the urine has been determined to exhibit an average sensitivity and specificity of 66% and 76%, respectively, for the diagnosis of PCa (Van Gils, 2007), and an AUC value of 0.693 was reported (Aubin, 2010). Elevated PCA3 scores have also been demonstrated to increase the probability of a positive repeat biopsy in men with one or two prior negative biopsy results (Haese, 2008). Although the specificity and sensitivity values of the PCA3 assays surpass the ones of PSA serum testing slightly, they are still unsatisfactory and hence, in the practical clinical use, the PCA3 test has not reached a wide acceptance.

Gleason score

The so-called Gleason grading system is used in combination with other parameters to predict PCa prognosis and guide therapy. The Gleason score is assigned by pathologists on the basis of microscopic features of prostate biopsies and reflects the differentiation status of the prostate tumour cells observed in the biopsy material (Gleason, 1977). Prostate cancers with low Gleason scores have better prognosis and the majority of them may not require treatment and should be monitored with active surveillance. However, the informative value is limited and the pinpointing of tumours that are aggressive and lethal despite having low Gleason scores remains a clinical challenge (Irshad, 2013).

Novel biomarkers

The TMPRSS2:ERG gene fusion is the most common genetic aberration in PCa, found in approximately 40% to 70% of patients. A sensitive TMPRSS2-ERG assay is available for clinical use but is unable to cover the TMPRSS2:ERG-negative PCa cases. Furthermore, a correlation of TMPRSS2:ERG with prognosis is controversial. In addition, a number of novel biomarkers have been proposed in the recent years as candidates for the use in diagnosis or prognosis of PCa but have not been introduced into clinical routine. This includes genetic as well as RNA biomarkers. A recent study to externally confirm the accuracy of the Select MDx test showed decreased performance than previously reported (Lendínez-Cano, 2021). The Mi-Prostate Score as well as ExoDx use the information of expression levels of the prostate-specific, non-coding RNA PCA3 and the fusion transcript TMPRSS2:ERG. Whereas, SelectMDx represents a qPCR-based assay measuring DLX1 and HOXC6 mRNA levels for detection of PCa. The three tests outlined emphasize their good clinical performance in predicting high-grade PCa (GS ≥ 7). In general, the inclusion of clinical information like age, findings of digital rectal examination (DRE) and previous biopsies further improve test performance (Cucchiara, 2017).

Summary

The strong focus on high-grade tumours of the tests described above improves test performance and may exclude low-risk PCa (equated to GS 6) for biopsy. However, it bears the risk to overlook at least some aggressive tumours. GS 6 PCa usually is described as non-threatening but it still is cancer and can progress to GS 7 or higher and metastasize. In this case different therapeutic approaches are required. GS 6 PCa patients are usually considered for active surveillance. It has been shown that more men on active surveillance showed disease progression than on radical therapy strategies. A test for early detection of PCa, prior to biopsy, shall identify patients who profit from further diagnosis, hence, exclusion of GS 6 tumours for test development may turn the test insensitive to an elevated risk subgroup of GS 6 PCa patients. It is debatable whether GS 6 PCa patients should be excluded for further invasive diagnosis a priori.
The aim of the current study was to identify new RNA biomarkers for early diagnosis of PCa (GS ≥ 6) in order to reduce unnecessary biopsies. This study did also not exclude patients with GS=6, thus the method of the invention may also detect early stages of PCa. In particular the method of the invention is aimed at a diagnostic differentiation of PCa tumours from BPH (benign prostatic hyperplasia). Previously published application WO 2015/082418 A1 also presents newly identified biomarkers for diagnosis of PCa, however the present application provides a superior method of diagnosis with improved reliability using different models and selections of biomarkers.

**Brief description of the invention**

Transcripts differentially expressed in tumour and control tissues were identified by Next Generation Sequencing of 64 samples of prostate cancer patients and controls; and validated by microarray and qRT-PCR analyses of 203 and 338 samples, respectively. From these samples a selection of potential RNA biomarkers was identified, which are suitable for use in the diagnosis of prostate cancer. Evaluation and optimization of the identified biomarkers was used to develop a diagnostic method which represents an improved and more reliable diagnosis of PCa. Essentially this diagnostic method includes stringent quality evaluation of patient samples and subsequent normalization of the RNA biomarkers to a selected and optimized set of reference RNAs. The normalized expression data of the RNA biomarkers is then analysed by an algorithm to determine the diagnosis.
The invention relates to a method for the diagnosis of prostate cancer comprising the steps of analysing the expression level of at least a nucleic acid selected from the group of SEQ ID NO: 2 and 20, wherein, if at least one of said nucleic acids is present and/or the expression level of at least one of said nucleic acids is above a threshold value, the sample is designated as prostate cancer positive.
In a preferred embodiment, the invention relates to a method for the diagnosis of prostate cancer comprising the steps of analysing the expression level of at least a nucleic acid according to SEQ ID NO: 2, SEQ ID NO: 19-20 and SEQ ID NO: 35-37 in a sample from a patient, wherein, if the expression level of said nucleic acid is above a threshold value, the sample is designated as prostate cancer positive.
In an alternative and equally preferred embodiment of the invention, the invention relates to a method for the diagnosis of prostate cancer comprising the steps of analysing the expression level of at least a nucleic acid according to SEQ ID NO: 1-3, SEQ ID NO: 20-34 and SEQ ID NO: 38-50 in a sample from a patient, wherein, if the expression level of said nucleic acid is above a threshold value, the sample is designated as prostate cancer positive.
In one embodiment, the invention relates to a nucleic acid that hybridizes under stringent conditions to one of the nucleic acids with SEQ ID NO: 1-3 or SEQ ID NO: 19-50, or any part thereof, or a nucleic acid that shares preferably at 85%, 90%, 91%, 92%, 93%, 94%, 95%, 96%, 97 %, 98% or 99% sequence identity with a nucleic acid according to any one of the nucleic acids according to SEQ ID NO: 1-3 or SEQ ID NO: 19-50.

The invention also relates to the use of a nucleic acid that hybridizes under stringent conditions to one of the nucleic acids with SEQ ID NO: 1-3 or SEQ ID NO: 19-50, or any part thereof, or a nucleic acid that shares preferably at 85%, 90%, 91%, 92%, 93%, 94%, 95%, 96%, 97 %, 98% or 99% sequence identity with a nucleic acid according to any one of the nucleic acids according to SEQ ID NO: 1-3 or SEQ ID NO: 19-50 for the diagnosis of prostate cancer.

The invention relates to a probe or primer, wherein the probe or primer is specific for a sequence of the group of SEQ ID NO: 1-131, preferably the specific sequences of the probe or primer are selected from the group comprising SEQ ID NO: 136-174.

The invention relates to the use of a nucleic acid with a sequence from the group of SEQ ID NO: 136-174 for the diagnosis of prostate cancer.

The invention relates to a nucleic acid with a sequence from the group of SEQ ID NO: 1-131, or the reverse complement thereof, or a nucleic acid that shares preferably at least 85%, 90%, 91%, 92%, 93%, 94%, 95%, 96%, 97 %, 98% or 99% sequence identity with a nucleic acid according to any one of the nucleic acids according to SEQ ID NO: 1-131.

The invention also relates to a kit for the diagnosis of prostate cancer comprising a nucleic acid that hybridizes under stringent conditions to the nucleic acid according to SEQ ID NO: 1-131, or a nucleic acid that shares preferably at least 85%, 90%, 91%, 92%, 93%, 94%, 95%, 96%, 97 %, 98% or 99% sequence identity with a nucleic acid according to any one of the nucleic acids according to SEQ ID NO: 1-131; and reagents for nucleic acid amplification and/or quantification and/or detection.

**Definitions**

The following definitions are provided for specific terms, which are used in the application text.

Unless otherwise defined, all technical and scientific terms used herein have the same meaning as commonly understood by one skilled in the art to which this technology belongs. Although all methods and materials similar or equivalent to those described herein may be used in practice or in testing the present technology, the preferred methods, devices and materials are now described.

Where an indefinite or a definite article is used when referring to a singular noun such as "a" or "an" or "the", this includes a plural form of that noun unless specifically stated. Vice versa, when the plural form of a noun is used it refers also to the singular form. For example, when biomarkers are mentioned, this is also to be understood as a single biomarker.

As used herein, "nucleic acid(s)" or "nucleic acid molecule" generally refers to any ribonucleic acid or deoxyribonucleic acid, which may be unmodified or modified. "Nucleic acids" include, without limitation, single- and double-stranded nucleic acids. As used herein, the term "nucleic acid(s)" also includes nucleic acids as described above that contain one or more modified bases. Thus, a nucleic acid with one or several backbone modifications for stability or for other reasons is a "nucleic acid". The term "nucleic acids" as it is used herein encompasses such chemically, enzymatically or metabolically modified forms of nucleic acids, as well as the chemical forms of nucleic acids characteristic of viruses and cells, including for example, simple and complex cells.

The term "transcript" relates to a nucleic acid produced by making a copy of a template nucleic acid. Included in this definition is the nucleic acid obtained through transcription by a polymerase of a template nucleic acid, for example a locus, to produce the new nucleic acid with a sequence complementary to the template. The term transcript also includes any nucleic acid further processed after transcription. Such further processing includes, but is not limited to, splicing, polyadenylation, editing, partial digestion, ligation, and labelling. The term also encompasses nucleic acids derived from the transcript through for example further transcription, reverse transcription, amplification or elongation. The transcript can correspond to any portion of the template nucleic acid, preferably to at least 20 nucleotides. The transcript also has a sequence identity with the template nucleotide or part of the template nucleotide, or the reverse complement of the template nucleic acid of at least 90%, preferably at least 95% and most preferably at least 99%.

The terms "locus (hg38)" and short "hg38" are used to provide references to specific genetic loci in the hg38 assembly of the human genome. The references are denoted in the format of e.g.: Chr2: 1,550,437-1,629,191" which relates to a sequence interval on the human chromosome 2 within the genome assembly.

"Sample molecules" or "individual sample templates" as used herein refers to any kind of nucleic acid molecules contained in a sample to by analysed, such as single-stranded or double-stranded DNA and/or RNA.

"Biomarker" within the present invention refers to any gene, gene transcript, gene transcript variant or non-coding genomic sequence which are predictive for diagnosis of PCa.

The terms "level" or "expression level" in the context of the present invention relate to the level at which a biomarker is present in a sample from a patient. The expression level of a biomarker is generally measured by comparing its expression level to the expression level of one or several reference or housekeeping genes in a sample for normalisation. The sample from the patient is designated as prostate cancer positive if the expression level of the biomarker exceeds the expression level of the same biomarker in an appropriate control (for example a healthy tissue) by a set threshold value. Expression levels are further calibrated for inter-run comparison using reference genes measured in calibrator samples.

The term, "analysing a sample for the presence and/or level of nucleic acids" or "specifically estimate levels of nucleic acids", as used herein, relates to the means and methods useful for assessing and quantifying the levels of nucleic acids. One useful method is for instance quantitative reverse transcription PCR. Likewise, the level of RNA can also be analysed for example by northern blot, next generation sequencing or after amplification by using spectrometric techniques that include measuring the absorbance at 260 and 280 nm.

The "amplification" of the individual sample templates refers to any kind of nucleic acid amplification method which results in the generation of multiples of the original template.

Analysis or examination herein refers to identifying whether or not amplification has taken place, identifying whether or not the target sequence lies between the primer regions and optionally, has the right length, identifying the amount of amplification product with a correct target sequence. Preferably in the method of the invention precise quantification of the amplification product is desired.

As used herein, the term "amplified", when applied to a nucleic acid sequence, refers to a process whereby one or more copies of a particular nucleic acid sequence is generated from a nucleic acid template sequence, preferably by the method of polymerase chain reaction (PCR). Other methods of amplification include, but are not limited to, ligase chain reaction (LCR), polynucleotide-specific based amplification (NSBA), loop-mediated isothermal amplification (LAMP), quantitative reverse transcription real-time PCR (qRT-PCR), quantitative (real-time) PCR (qPCR), droplet digital PCR, digital PCR, or any other amplification method known in the art.

The term "correlating", as used herein in reference to the use of diagnostic and prognostic marker(s), refers to comparing the presence or amount of the marker(s) in a sample from a patient to its presence or expression level in a sample from a person known to suffer from or at risk of suffering from a given condition. A marker expression level in a patient sample can be compared to a level known to be associated with a specific diagnosis.

As used herein, the terms "diagnosis" or "diagnostic" refer to the identification of the disease, in this case prostate cancer, at any stage of its development, and also includes the determination of predisposition of a subject to develop the disease.

The "labeling" of the DNA or RNA strands can be realized by associating or incorporating any kind of marker detectable by conventional imaging techniques, e.g. a fluorescent marker.

As used herein, the term "fluorescent dye" refers to any chemical that absorbs light energy of a specific wavelength and re-emits light at a different wavelength.

Application of a fluorescence dye for target detection implies either use of intercalating dyes in a PCR reaction to detect formation of double-stranded DNA molecules, or labelling primers or probes with suitable fluorophore molecules to observe presence of formation of nucleic acid molecules with complementary target sequences in dependence on the detected fluorescence signals of the fluorophore molecules. By using different fluorophores with distinct emission and excitation spectra it is possible to combine more than one detection system into one PCR reaction (multiplex PCR) and to separately detect the fluorescence signals. Potential labelling molecules include but are not limited to fluorescence dye or chemiluminescence dye in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like. In the context of the present invention, chemiluminescence based assays comprise the use of dyes, based on the physical principles described for chemiluminescent materials in Kirk-Othmer, Encyclopedia of chemical technology, 4th ed., executive editor, J. I. Kroschwitz; editor, M. Howe-Grant, John Wiley & Sons, 1993, vol.15, p. 518-562, incorporated herein by reference, including citations on pages 551-562. Preferred chemiluminescent dyes are acridiniumesters.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use. If the kit contains nucleic acids, the kit may also comprise synthetic or non-natural variants of said nucleic acids. A synthetic or non-natural nucleic acid is to be understood as a nucleic acid comprising any chemical, biochemical or biological modification, such that the nucleic acid does not appear in nature in this form. Such modifications include, but are not limited to, labelling with a fluorescent dye or a quencher moiety, a biotin tag, as well as modification(s) in the backbone of a nucleic acid, or any other modification that distinguishes the nucleic acid from its natural counterpart. The same applies also to other natural compounds such as proteins, lipids and the like.

The term "patient" as used herein refers to a living human or non-human organism that is receiving medical care or that should receive medical care due to a disease, or is suspected of having a disease. This includes persons with no defined illness who are being investigated for signs of pathology. Thus the methods and assays described herein are applicable to both, human and veterinary disease.

The term "primer" as used herein, refers to an nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. Preferably, primers have a length of from about 15-100 bases, more preferably about 20-50, most preferably about 20-40 bases. The factors involved in determining the appropriate length of primer are readily known to one of ordinary skill in the art. Optionally, the primer can be a synthetic element, in the sense that it comprises a chemical, biochemical or biological modification. Such modifications include, but are not limited to, labelling with a fluorescent dye or a quencher moiety, or a modification in the backbone of a nucleic acid, or any other modification that distinguishes the primer from its natural nucleic acid counterpart.

The term "probe" refers to any element that can be used to specifically detect a biological entity, such as a nucleic acid, a protein or a lipid. Besides the portion of the probe that allows it to specifically bind to the biological entity, the probe also comprises at least one modification that allows its detection in an assay. Such modifications include, but are not limited to labels such as fluorescent dyes, quenchers, a specifically introduced radioactive element, or a biotin tag. The probe can also comprise a modification in its structure, such as a locked nucleic acid.

The term "sample" as used herein refers to a sample of bodily fluid or tissue obtained for the purpose of diagnosis, prognosis, or evaluation of a subject of interest, such as a patient. In addition, one of skill in the art would realize that some test samples would be more readily analysed following a fractionation or purification procedure, for example, separation of whole blood into serum or plasma components.

Thus, in a preferred embodiment of the invention the sample is selected from the group comprising prostate tissue, biopsy material, lymph nodes, urine, ejaculate, blood, blood serum, blood plasma, circulating tumour cells in blood or lymph, any tissue suspected of containing metastases as well as any source that may contain prostate tumour cells or parts thereof, including vesicles like exosomes, micro vesicles, and others as well as free or protein-bound RNA molecules derived from prostate tumour cells. Preferably, the sample is a blood sample, most preferably a serum sample or a plasma sample. Importantly, urine (particularly after digital rectal examination) and ejaculate belong to the most preferable samples. Tissue samples may also be biopsy material or tissue samples obtained during surgery.

The term "area under the curve (AUC)" as used herein describes the area under the curve of a receiver operating characteristic (ROC) or ROC curve. The AUC relates to how specific and sensitive a biomarker is. A perfect marker (AUC=1.0) would yield a point in the upper left corner or coordinate (0,1) of the ROC space, representing 100% sensitivity (no false negatives) and 100% specificity (no false positives).

The term "p-value" relate to the probability of obtaining the observed sample results (or a more extreme result) when the null hypothesis is actually true, i.e. there are no differences between means for groups. The smaller the p-value, the higher the likelihood that the alternative hypothesis explains the observed results better than the null hypothesis.

The term "adjusted p-value" refers to p-values which have been adjusted for multiple comparisons according to Benjamini & Hochberg. The method applied is detailed in the experimental section.

**Detailed description of the invention**

The invention describes a method of diagnosis of prostate cancer. This method comprises analysing a sample taken from a patient and specifically determining the level of a biomarker or a combination of biomarkers in said patient sample. The result is then correlated to a threshold value and in the case where it is above that threshold value, said patient sample is designated as prostate cancer positive. Both the calculated score of the biomarker or biomarkers and the threshold value are obtained according to a machine-learning model.

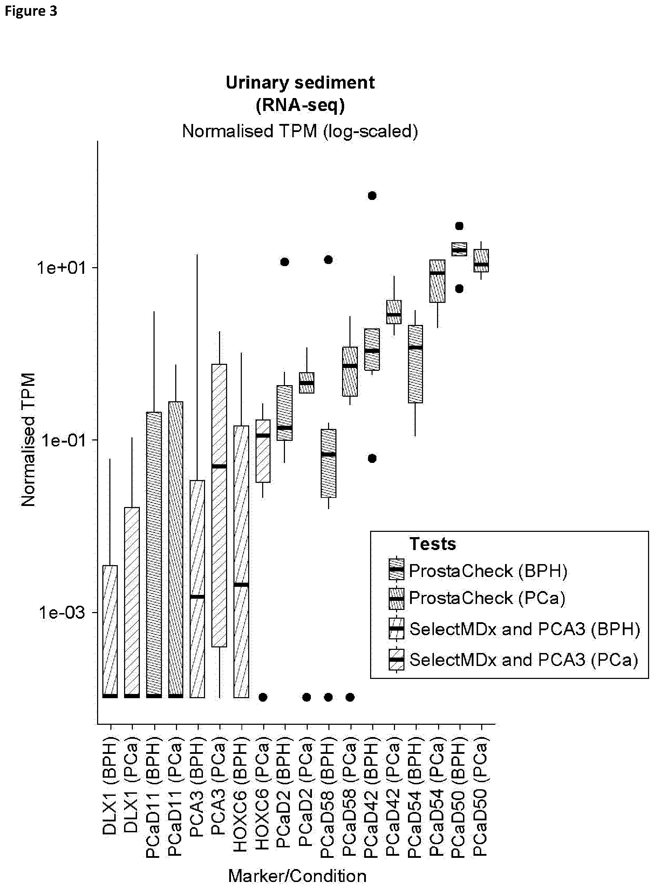

The experimental data of this invention show an impressive sensitivity and specificity of the newly identified biomarkers (ProstaCheck; including 2 distinct assay models), which clearly outperform current assays on the market as seen by the increased AUC-value for the ProstaCheck models (Progensa PCA3, SelectMDx; see Table 1). Figure 3 also presents a comparison of the newly identified biomarkers in comparison to commercially available SelectMDx and PCA3 assays, this figure also clearly indicated that the newly identified biomarkers are superior to the state of the art assays.

Diagnostic assays based on these new biomarkers therefore decrease the high false-positive rates of current assays and thereby help to avoid unnecessary invasive prostate biopsies. In addition, the method of the invention does not exclude men with GS=6, which were also included in the experimental data sets, thus enabling early diagnosis of prostate cancer by the method of the invention. Further, the method of the invention in particular includes criteria for stringent assessment of sample quality prior to application of the diagnostic assay and necessary normalization and calibration procedures for the detected biomarkers. Sample quality assessments, normalization and calibration are significant elements of the method of the invention, which ensures reliable and reproducible results.

Table 1: Direct comparison of ProstaCheck with state of the art PCa assays Progensa PCA3 and SelectMDx in urinary sediment samples using RT-qPCR

| Method | AUC [95% CI] | Specificity at 90% sensitivity | Number of markers |
|---|---|---|---|
| ProstaCheck Model 1 | 0.817 [0.73-0.91] | 0.512 | 4 biomarkers 5 reference markers |
| ProstaCheck Model 2 | 0.829 [0.74-0.92] | 0.605 | 6 biomarkers 5 reference markers |
| Progensa PCA3* | 0.778 [0.70-0.86] | 0.467 | 1 biomarker 1 reference marker |
| SelectMDx** | 0.76 | - | 2 biomarkers 1 reference marker |

(*) AUCs assessed at same patients in independent validation cohort (n=87). (**) AUC taken from Cui et al. "Evaluation of prostate cancer antigen 3 for detecting prostate cancer: a systematic review and meta-analysis", Scientific Reports, May 2016

Apart from prostate cells, other cells may be present in urine or urinary sediment samples. To ensure that the biomarker assay of invention is not negatively affected by the presence of varying amounts of non-prostate cells the detected expression of promising biomarker candidate genes was correlated with expression of cell-specific reference genes. Typically urine or urinary sediment samples may contain leukocytes, kidney cells or bladder cells apart from cells of the prostate. Thus cell-specific reference genes for these cell types were selected, i.e. CD45 (leukocytes), UMOD (kidney cells), NPHS2 (kidney cells) and UPK2 (bladder cells). Biomarker candidates were only further considered if their expression did not correlate with expression of the mentioned cell-specific reference genes. Thus, the biomarkers of the present invention represent expression within prostate cells.

The term "urinary sediment", refers to cells, cell debris and other sedimented material obtained by centrifugation of urine.

The combination of biomarkers and the calculation of threshold values as well as the calculation of sample analysis for diagnosis is achieved by a multivariate statistical model, which is obtained by a machine learning approach using training set samples of different PCa stages in comparison to BPH (benign prostatic hyperplasia). The newly identified biomarkers of the invention were found to be located in the genomic regions of the following genes: CPNE4 (biomarkers: PCaD2 [SEQ ID NO: 1]; PCaD4 [SEQ ID NO: 2]; PCaD52 [SEQ ID NO: 21-34]), TAPIR novel ncRNA (biomarker PCaD11 [SEQ ID NO: 3]), HOXB13 (biomarker PCaD42 [SEQ ID NO: 19]), PCAT14 (biomarker PCaD50 [SEQ ID NO: 20]), MSMB (biomarker PCaD54 [SEQ ID NO: 35-37]) and FOLH1 (biomarker PCaD58 [SEQ ID NO: 38-50]). Some of the listed biomarkers are situated in exons of known transcript variants of their respective genes, other are located within introns according to current annotation and therefore may relate to either new exons of yet unknown transcript variants or novel genes encoding for non-coding RNA. As reference previously described and commonly applied PCa biomarker PCA3 was also measured (biomarker PCaD30 [SEQ ID NO: 132-135]) by either custom designed primers or by commercially available Progensa PCA3 test. Table 2 provides an overview over the newly identified biomarkers for diagnosis of prostate cancer as well as a statistical evaluation of these biomarkers. The performance of these biomarkers is shown in table 2 and in figure 3.

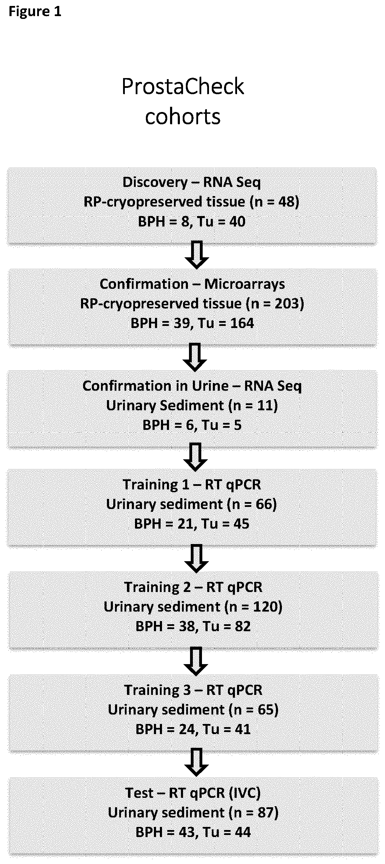

These biomarkers were identified within the "discovery cohort" (see figure 1" using negative binominal log-linear models which were fitted to the observed data for each transcript or gene and regression coefficients were identified by a likelihood ratio test. As indicated in table 1 the identified new biomarkers were combined into diagnostic assay within two different logical models. These models were obtained by machine-learning. The scheme for identification of biomarkers, evaluation, validation and machine-learning is shown in figure 1. These models are logistic regression models which were fitted to the observed data for each biomarker and to receive regression coefficients. The first logical model combines biomarkers PCaD4, PCaD42, PCaD50 and PCaD54. The regression coefficients of these biomarkers in this model are: -0.03104, 0.04972, -0.13662 and -0.14546, respectively. The second logical model combines biomarkers PCaD2, PCaD4, PCaD11, PCaD50, PCaD52 and PCaD58. The regression coefficients of these biomarkers in this model are: -0.06262, -0.01419, - 0.03030, -0.05290, -0.03138 and -0.04455, respectively. Thus, both models contain PCaD4 and PCaD50, these two biomarkers represent an essential minimal component of both models. Further, these biomarkers may also as an alternative embodiment of the invention be combined on their own into a further model. However, inclusion of the additional biomarkers of the two described models improves the predictive value of the diagnostic assays.

All applied models further involve the use of reference biomarkers. These reference biomarkers are used for quality control of the samples as well as for normalization of the biomarker measurements. Within the assay models of the invention the following reference markers are used: KLK2 (biomarker PCaD91 [SEQ ID NO: 51-73]), KLK3 (biomarker PCaD32 [SEQ ID NO: 4-18]), EMC7 (biomarker PCaD92 [SEQ ID NO: 74-77]), RAB7A (biomarker PCaD93 [SEQ ID NO: 78-95]) and VCP (biomarker PCaD94 [SEQ ID NO: 96-131]).

Thus, the invention relates to a group of nucleic acids, the sequences of these nucleic acids comprise SEQ ID NOs 1 to 131. The relevant characteristics of these sequences are listed below in table 2 with further information regarding statistical analysis of individual biomarkers. The sequences are provided separately in the sequence listing. These sequences represent the selected biomarkers and selected reference markers (reference genes) of the present diagnostic method. Performing the present diagnostic method involves detection of at least one of the listed biomarkers and at least one of the listed reference markers and using the detected expression levels to determine the patient's diagnosis regarding PCa.

Table 2: List of biomarkers and reference genes: Each biomarker and reference gene is listed together with the gene annotations and with statistical evaluation of each biomarkers. For several biomarkers and reference genes multiple transcript variants are known. Each transcript variant corresponds to a SEQ ID NO which are listed for each biomarker and reference gene, sequences and Ensembl Transcript IDs for each SEQ ID NO are presented in the sequence listing and in table 6.

| Marker label(SEQ ID NO) | Gene symbol | Description | Ensembl Gene ID (Gencode V41) | Type* | Biotype** | PPV in IVC*** | NGV in IVC*** | AUC in IVC*** | p-value (U-test) in IVC*** |
|---|---|---|---|---|---|---|---|---|---|
| PCaD2 (SEQ ID NO:1) | CPNE4 | Copine 4 (synonyms: COPN4, CPN4) Biomarker | ENSG00000196353 | intron | non coding | 0.775 | 0.75 | 0.76 | 0.0000247 |
| PCaD4 (SEQ ID NO:2) | CPNE4 | Copine 4 (synonyms: COPN4, CPN4) Biomarker | ENSG00000196353 | intron | non coding | 0.698 | 0.818 | 0.789 | 0.00000171 |
| PCaD 11 (SEQ ID NO:3) | TAPIR | Novel ncRNA Biomarker | ENSG00000203635 | 1 transcript variant | non coding | 0.806 | 0.706 | 0.792 | 0.0000011 |
| PCaD32 (SEQ ID NO:4-18) | KLK3 | Kallikrein related peptidase 3 (synonyms: APS, KLK2A1, PSA, hK3) Reference marker | ENSG00000142515 | 15 transcript variants | coding | - | - | - | - |
| PCaD42 (SEQ ID NO: 19) | HOXB13 | Homeobox B13 (synonym: HPC9, PSGD) Biomarker | ENSG00000159184 | 1 transcript variant | coding | 0.794 | 0.679 | 0.762 | 0.0000157 |
| PCaD50 (SEQ ID NO:20) | PCAT14 | Prostate cancer associated transcript 14 Biomarker | ENSG00000280623 | intron | non coding | 0.81 | 0.762 | 0.807 | 0.000000455 |
| PCaD52 (SEQ ID NO:21-34) | CPNE4 | Copine 4 (synonyms: COPN4, CPN4) Biomarker | ENSG00000196353 | 14 transcript variants | coding | 0.789 | 0.717 | 0.801 | 0.00000078 |
| PCaD54 (SEQ ID NO:35-37) | MSMB | Microseminoprotein beta (synonyms: HPC13, IGBF, MSP, MSPB, PN44, PRPS, PSP, PSP-94, PSP57, PSP94) Biomarker | ENSG00000263639 | 3 transcript variants | coding | 0.757 | 0.68 | 0.758 | 0.0000202 |
| PCaD58 (SEQ ID NO:38-50) | FOLH1 | Folate hydrolase 1 (synonyms: FGCP, FOLH, GCP2, GCPII, NAALAD1, PSM, PSMA, mGCP) Biomarker | ENSG00000086205 | 13 transcript variants | coding | 0.756 | 0.717 | 0.795 | 0.00000082 |

| Marker label (SEQ ID NO) | Gene symbol | Description | Ensembl Gene ID (Gencode V41) | Type* | Biotype** | PPV in IVC*** | NGV in IVC*** | AUC in IVC*** | p-value (U-test) in IVC*** |
|---|---|---|---|---|---|---|---|---|---|
| PCaD91 (SEQ ID NO:51-73) | KLK2 | Kallikrein related peptidase 2 (synonyms: KLK2A2, hGK-1, hK2) Reference marker | ENSG00000167751 | 23 transcript variants | coding | - | - | - | - |
| PCaD92 (SEQ ID NO:74-77) | EMC7 | ER membrane protein complex subunit 7 (synonyms: C11orf3, C15orf24, HT022, ORF1-FL1) Reference marker | ENSG00000134153 | 4 transcript variants | coding | - | - | - | - |
| PCaD93 (SEQ ID NO:78-95) | RAB7A | RAB7, member RAS oncogene family (synonyms: Rab7a) Reference marker | ENSG00000075785 | 18 transcript variants | coding | - | - | - | - |
| PCaD94 (SEQ ID NO: 96-131) | VCP | Valosin containing protein (synonyms: CDC48, FTDALS6, TERA, p97) Reference marker | ENSG00000165280 | 36 transcript variants | coding | - | - | - | - |
| PCaD30 (SEQ ID NO: 132-135) | PCA3 | Prostate cancer associated 3 (synonyms: DD3, NCRNA00019, PCAT3, PRUNE2-AS1) Biomarker | ENSG00000225937 | 4 transcript variants | non coding | 0.791 / 0.761 / 0.741# | 0.767 / 0.724 / 0.717# | 0.821 / 0.805 / 0.778# | 7.27E-08 / 0.00000715 / 2.43E-08 # |

PPV: positive predictive value; NPV: negative predictive value; AUC: area under the curve; IVC: independent validation cohort (see Figure 1); *: sequences and transcript IDs for each transcript variant are provided in the sequence listing and in table 6 under the corresponding SEQ ID NOs of biomarkers and reference genes, if no known transcript variant is given, than the sequence of the corresponding gene ID is entered into the respective SEQ ID NO; **: sequences are either protein coding or non-coding; ***: statistical evaluation only applicable for biomarker not reference markers; #: Prostacheck PCA3 / Progensa PCA3 assay (incl. QC filter) / Progensa PCA3 assay (excl. QC filter)

For detection of the biomarkers of table 2 specific primer and probes were designed. As the person skilled in the art is aware there are usually numerous possible design options available to obtain specific and reliable primers and probes for a sufficiently long target sequence. Hence, any primer or probe which is suited for reliable and specific detection of the biomarkers and reference genes as listed in table 2 is part of the present invention. The inventors specifically designed and validated the primers and probes listed in table 3. As stated above these primers and probes are to be understood as possible examples, other primers and probes are available and the skilled person in the art in aware on how to design and validate these. Thus, table 3 does not represent a comprehensive list of all primers and probes of the invention. Use of the primers and probes of table 3 for detection of the biomarkers and reference genes is a preferred embodiment of the invention.

Table 3: List of primers (FW, RE), probes (PR) and amplicon positions used for amplification and detection of target biomarkers and reference genes of table 2.

| Biomarker label (SEQ ID NO) | Target gene | Oligo Type | Sequence (5'-3') | Amplicon position* |
|---|---|---|---|---|
| PCaD2 (SEQ ID NO: 136) | CPNE4 (intron) | FW | GCAAATCAAAGCAGGTGAGA | hg38: chr3: 132241508-132241581 |
| PCaD2 (SEQ ID NO: 137) | CPNE4 (intron) | RE | ACTATGCCTCACCACCATCA | |
| PCaD2 (SEQ ID NO: 138) | CPNE4 (intron) | PR | AGGCTACACCCAAACCCTGTCCTTC | |
| PCaD4 (SEQ ID NO: 139) | CPNE4 (intron) | FW | ACCTTGGGCCTCTGCTAAA | hg38: chr3: 132259812-132259946 |
| PCaD4 (SEQ ID NO: 140) | CPNE4 (intron) | RE | TTCCTGCTGACTTCCACTTG | |
| PCaD4 (SEQ ID NO: 141) | CPNE4 (intron) | PR | ACTCAATGGGTGACACTCACAGGACA | |
| PCaD11 (SEQ ID NO: 142) | TAPIR | FW | ATTGACCGCAGAGCTACCTT | hg38: chr2: 1621114-1621206 |
| PCaD11 (SEQ ID NO: 143) | TAPIR | RE | GCATGAAAGTGGACCAACAC | |
| PCaD11 (SEQ ID NO: 144) | TAPIR | PR | TCTGGATGCCACACGGCCTC | |
| PCaD32 (SEQ ID NO: 145) | KLK3 | FW | CAAGCTTACCACCTGCACC | hg38: chr19: 50854920-50856387 |
| PCaD32 (SEQ ID NO: 146) | KLK3 | RE | TGGGCAGCTGTGAGGAC | |
| PCaD32 (SEQ ID NO: 147) | KLK3 | PR | CTGCGGCGGTGTTCTGGTGCA | |
| PCaD42 (SEQ ID NO: 148) | HOXB13 | FW | AACCTGGCCCAGTCATAATC | hg38: chr17: 48726484-48726562 |
| PCaD42 (SEQ ID NO: 149) | HOXB13 | RE | GCTAACGATCATGGCAGCTA | |
| PCaD42 (SEQ ID NO: 150) | HOXB13 | PR | CATCCTGACAGTGGCAATAATCACGA | |
| PCaD50 (SEQ ID NO: 151) | PCAT14 (intron) | FW | CCCAACCGCTGGTAGTTTAT | hg38: chr22: 23539571-23539689 |
| PCaD50 (SEQ ID NO: 152) | PCAT14 (intron) | RE | GTATGGTGCCGTGCTATTTG | |

(continued)

| Biomarker label (SEQ ID NO) | Target gene | Oligo Type | Sequence (5'-3') | Amplicon position* |
|---|---|---|---|---|
| PCaD50 (SEQ ID NO: 153) | PCAT14 (intron) | PR | ATACCGGCTGCCAACCGAGC | |
| PCaD52 (SEQ ID NO: 154) | CPNE4 | FW | GGAAACTGGAAAGACACACG | hg38: chr3: 132037670-132039575 |
| PCaD52 (SEQ ID NO: 155) | CPNE4 | RE | AAAGGAAGCTTCAGCTCTGG | |
| PCaD52 (SEQ ID NO: 156) | CPNE4 | PR | TCCAGAAACCCGGCGAGTGA | |
| PCaD54 (SEQ ID NO: 157) | MSMB | FW | GGAGTCCTGCTTATCACAATGA | hg38: chr10: 46039986-46046255 |
| PCaD54 (SEQ ID NO: 158) | MSMB | RE | TCCTGGTTGAATCTCCTGGA | |
| PCaD54 (SEQ ID NO: 159) | MSMB | PR | CGTTGTGATCTTTGCCACCT | |
| PCaD58 (SEQ ID NO: 160) | FOLH1 | FW | GAAAGCAAAGTGGACCCTTC | hg38: chr11: 49146715-49146863 |
| PCaD58 (SEQ ID NO: 161) | FOLH1 | RE | CATACCACACAAATTCAATACGG | |
| PCaD58 (SEQ ID NO: 162) | FOLH1 | PR | TTCACAGTGCAGGCAGCTGCA | |
| PCaD91 (SEQ ID NO: 163) | KLK2 | FW | TGTGTGCTGGGCTCTGG | hg38: chr19: 50876968-50878495 |
| PCaD91 (SEQ ID NO: 164) | KLK2 | RE | GCAGGCTTTTCAGGCAGG | |
| PCaD91 (SEQ ID NO: 165) | KLK2 | PR | TCTGGGGGTCCACTTGTCTGTAATGG | |
| PCaD92 (SEQ ID NO: 166) | EMC7 | FW | AGTGATCCTGACATGAGACGG | hg38: chr15: 34084364-34088073 |
| PCaD92 (SEQ ID NO: 167) | EMC7 | RE | TGTTTTACTGCTGCCGCTGC | |
| PCaD92 (SEQ ID NO: 168) | EMC7 | PR | TGCTGAATTCCAACCATGAGTTGCC | |
| PCaD93 (SEQ ID NO: 169) | RAB7A | FW | AGGCGTTCCAGACGATTGC | hg38: chr3: 128807634-128813446 |
| PCaD93 (SEQ ID NO: 170) | RAB7A | RE | CTGTGCTCAACTCTCACTGC | |
| PCaD93 (SEQ ID NO: 171) | RAB7A | PR | ACCGGGCCAAGGCCTCGGCA | |
| PCaD94 (SEQ ID NO: 172) | VCP | FW | TTTGCCCAGACCCTTCAGC | hg38: chr9: 35057089-35057419 |
| PCaD94 (SEQ ID NO: 173) | VCP | RE | CAGCTCACTGCACGCTGG | |
| PCaD94 (SEQ ID NO: 174) | VCP | PR | AGGGCAGTGGAGGCGGCACAGG | |

(continued)

| Biomarker label (SEQ ID NO) | Target gene | Oligo Type | Sequence (5'-3') | Amplicon position* |
|---|---|---|---|---|
| PCaD30 (SEQ ID NO: 175) | PCA3 | FW | CACACACACACAGGAAGCACAA | hg38: chr9: 76783854-76784201 |
| PCaD30 (SEQ ID NO: 176) | PCA3 | RE | AATGTCCTTCCCTCACAAGC | |
| PCaD30 (SEQ ID NO: 177) | PCA3 | PR | ATGAGCCTCGCCCTGTGCCT | |
| FW: forward primer; RE: reverse primer; RP: probe; *: position of amplicon as defined by respective forward and reverse primers on reference human genome assembly hg38 | | | | |

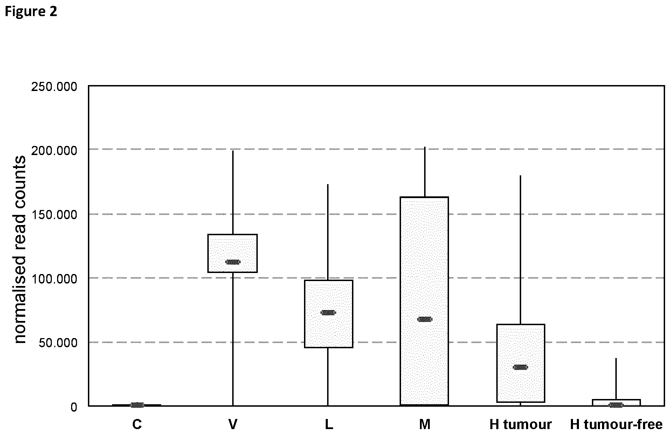

The biomarker PCA3 is routinely used for prostate carcinoma (PCa) diagnosis. As expected therefore, PCA3 expression levels were indicative of PCa in the subjects tested by next generation sequencing by the inventors (fig. 2). However, it was found that the biomarker had its highest expression level in very low risk tumours (V) and decreased as the risk factor of tumours grew. This finding makes PCA3 an unreliable marker for medium- and high-risk tumours and shows the need for better prostate cancer biomarkers.

Hence, the invention relates to a method for the diagnosis of prostate cancer comprising the steps of analysing the expression level of the nucleic acid according to SEQ ID NO: 1-3 and 19-50, wherein, if at least one of said nucleic acids is present and/or the expression level of at least one of said nucleic acids is above a threshold value, the sample is designated as prostate cancer positive.

In an alternative embodiment, analysing the expression level of a nucleic acid means analysing the reverse complement or the cDNA of the nucleic acid. The experimental results demonstrate high specificity and sensitivity of the novel biomarkers for the detection of PCa as shown in figure 3.

In a preferred embodiment, the sample is selected from the group comprising prostate tissue, biopsy material, lymph nodes, urine, ejaculate, blood, blood serum, blood plasma, circulating tumour cells in blood or lymph, any tissue suspected of containing metastases as well as any source that may contain prostate tumour cells or parts thereof, including vesicles like exosomes, micro vesicles, and others as well as free or protein-bound RNA molecules derived from prostate tumour cells or parts thereof. More preferably the sample is urine, even more preferably the sample is urinary sediment, still more preferably the sample is urine obtained from a patient after a digital rectal examination and most preferably the sample is urinary sediment obtained from a patient after a digital rectal examination.

Measurement of the RNA biomarkers of the invention can be done by any method suited to specifically estimate RNA levels, e.g. PCR-based methods like qRT-PCR. The assays can be applied for early diagnosis (screening) of PCa, for predicting the aggressiveness of the tumours (prognosis), and/or for aiding the choice of therapy.

In one aspect of the invention, the expression level of a transcript of the biomarker nucleic acids according to SEQ ID NO: 1-3 and 19-50, is compared to the expression level of one or several other gene transcripts in the sample, which are used as reference genes. Thus, the expression levels of the biomarker genes can be normalized according to the expression levels of the reference genes. Examples of suitable reference genes are shown below in Table 4, however this list is not exhaustive, the person skilled in the art is aware of further possible candidates.

Table 4: Potential reference genes

| General reference genes | Synonyms |
|---|---|
| EMC7 - ER membrane protein complex subunit 7 | C11orf3, C15orf24, HT022, ORF1-FL1 |
| GAPDH - Glyceraldehyde 3-phosphate dehydrogenase | G3PD, GAPD, HEL-S-162eP |
| GPI -glucose-6-phosphate isomerase | AMF, GNPI, NLK, PGI, PHI, SA-36, SA36 |
| HMBS - hydroxymethylbilane synthase | PBG-D, PBGD, PORC, UPS |
| HPRT1 - hypoxanthine phosphoribosyltransferase 1 | HGPRT, HPRT |
| RAB7A - RAB7A, member RAS oncogene family | Rab7, CMT2B, PRO2706 |
| TBP -Tata box binding protein | GTF2D, GTF2D1, HDL4, SCA17, TBP1, TFIID |
| VCP - valosin containing protein | CDC48, FTDALS6, TERA, p97 |
| **Prostate specific reference genes** | |
| ACPP - acid phosphatase, prostate | ACP3, FRAP, LAP, PAP, PPAL |

(continued)

| Prostate specific reference genes | |
| --- | --- |
| KLK2 - kallikrein related peptidase 2 | KLK2A2, hGK-1, hK2 |
| KLK3 - kallikrein related peptidase 3 | APS, KLK2A1, PSA, hK3 |
| RDH11 - retinol dehydrogenase 11 | ARSDR1, CGI82, HCBP12, MDT1, PSDR1, RALR1, RDJCSS, SCALD, SDR7C1 |

In another aspect of the invention the method of the invention involves stringent quality control criteria for inclusion of subjects' samples into diagnostic tests. The inventors of the present invention surprisingly found that a three-tier quality control protocol ensures reliable and accurate diagnostic tests. The first tier involves the careful review of amplification curves regarding appropriate sigmoid curve shape and sufficiently large amplification as well as sufficient amplification efficiency. Only amplification of sufficient quality and reliability can be validly included into the diagnostic analysis. This includes confirming a sigmoid shape of the amplification curve, i.e. a difference of the asymptotes of at least 1.5, and a minimal amplification efficiency of the qPCR of at least 1.5.

Furthermore, quality control (QC) of test samples involves a first QC filter. The first filter tests for sufficient minimal amount of cells and for sufficient minimal amount of prostate cells within the sample (urinary sediment sample). The first QC filter comprises determining the expression of a selection of generally applicable reference genes in combination with at least one prostate specific reference gene. The inventors tested different possible reference genes for applicability within the method of the invention. The person skilled in the art is aware that commonly any gene with consistent and reliably detectable expression which is stable across at least most cellular conditions and cultivation states, is a potential candidate for a reference gene. Although suitability of candidate for a particular application always needs to be tested and appropriately established. Suitable approaches for selection of candidate genes and testing of suitability is within the common knowledge of the person skilled in the art. Suitable candidates for reference genes can be selected from but are not limited to the group of genes as listed in table 4. A number of genes were tested as candidate reference genes, wherein the following genes were tested as general reference genes: EMC7, GAPDH, GPI, HMBS, HPRT1, RAB7A, TBP and VCP and the following genes were tested as prostate specific reference genes: ACPP, KLK2, KLK3 and RDH11 (table 4). All these reference genes may be used in alternative embodiments of the invention. In a preferred embodiment of the invention the reference genes of the first QC filter are selected from the group comprising general reference genes EMC7, RAB7A and VCP, and prostate specific reference gene KLK2. Further relevant information on these reference markers is provided in tables 2 and 3.

For a sample to pass the first QC filter all of the four tested reference genes (EMC7, RAB7A, VCP, KLK2) have to show expression levels above the threshold level. The inventors determined the following threshold levels: for EMC7 a sample passes the threshold if the expression level is at least smaller than a $c_q$-value of 28 (all $c_q$-values of this invention are calculated according to $Cy_0$ method-Guescini, 2008; however alternative approaches for calculation of $c_q$-values are well known in the art and are equally valid for the present invention), for RAB7A the threshold is if the expression level is at least smaller than a $c_q$-value of 30, for VCP the threshold is if the expression level is at least smaller than a $c_q$-value of 28, for KLK2 the threshold is if the expression level is at least smaller than a $c_q$-value of 28.

For further improved reliability of the first QC filter, expression levels are determined in triplicate amplification reactions. Each test is only suited for analysis if either the maximal difference of $c_q$-value between individual triplicates is at most 0.5 or if at least the maximal difference of $c_q$-value between two triplicates is at most 0.25. If a sample does not pass the first QC filter the sample is invalid and excluded from further analysis. The described first QC filter is then combined with a second QC filter.

The second QC filter is applied after normalization and calibration of the observed diagnostic results. This aspect of the quality control of the invention ensures that only reliable and reproducible data without large variance are included in the analysis. The person skilled in the art is aware that this aspect of the invention depends on the method and device used for quantifying the expression levels of the biomarkers and it is within the skill of the skilled person of the art to accordingly adjust these quality control methods to a different method than RT-qPCR. Also minimal variations in the number of replicates (4, 5 or 6 replicates instead of 3) or slight variations of the cut-off values will only minimally affect this quality control test and are therefore also included in the present invention.

Similarly, to the reference genes the selected biomarkers were also determined in triplicate amplification reactions for each sample to ensure reliable diagnostics. If the $c_q$-value of at least two triplicates of a sample is at least smaller than a selected threshold value, the respective test is only suited for analysis if either the maximal difference of $c_q$-value between individual triplicates is at most 0.5 or if at least the maximal difference of $c_q$-value between two triplicates is at most 0.25. If a sample does not pass this test the sample is invalid and excluded from further analysis. Alternatively, if the $c_q$-value of at least two triplicates of each test is not at least smaller than a selected threshold value or no threshold was preselected, the respective test may still be analysed using only valid $c_q$-values. A valid $c_q$-value in the meaning of the present invention refers to a $c_q$-value of a sample that has passed all prior QC checks. However, in this alternative case an increased variance of results can be expected.

As stated above the person skilled in the art is aware that this quality control may be adapted to different methods with slight variations, which are also included in the present invention.

The measured $c_q$-values of valid replicates for each sample, i.e. replicates which passed all prior QC steps, are used for diagnostic analysis. Diagnostic analysis comprises calculation of arithmetic means for each set of technical replicate measurements (formula 1).

$$Cq_{jk} = \overline{Cq_{jk}} = \frac{\sum_{i=1}^{N} Cq_{ijk}}{N}$$

Formula 1: Mean $c_q$-value calculation; N: number of technical replicates; i: count of technical replicates; j: counter of genes; k: counter of samples

For a relative quantification, an average expression over all samples on the same plate is calculated for each gene separately (formula 2). Reference samples for inter-run calibration are excluded from this calculation.

$$Cq_{reference,j} = \overline{Cq_j} = \frac{\sum_{k=1}^{s} Cq_{jk}}{s}$$

Formula 2: Mean $c_q$-value calculation per gene for all samples; s: number of samples; k: counter of samples; j: counter of genes

The difference of mean $c_q$-values (formula 1) and the average expression for the respective gene ($Cq_{reference,j}$, formula 2) is calculated and represents the relative quantification of the expression level of biomarkers or reference genes in the respective samples (formula 3).

$$RQ_{jk} = \Delta Cq_{jk} = Cq_{jk} - Cq_{reference,j}$$

Formula 3: Calculation of relative quantification: RQ; j: counter of genes; k: counter of samples

These relative quantifications are normalized according to standard practice in the field by first calculating a normalization factor. The normalization factor is calculated as the arithmetic mean of the relative quantifications of the reference genes (formula 4).

$$NF_k = \frac{\sum_{p=1}^{f} RQ_{pk}}{f}$$

Formula 4: Calculation of normalization factor: NF; f: number of reference genes; p: counter of reference genes; k: counter of samples

In a preferred embodiment the used reference genes are the general reference genes of the first QC filter. Nevertheless, the person skilled in the art is aware, how to select and validate a possible alternative set of reference genes. The thus obtained normalization factor is subsequently used to calculate normalized relative quantification of the selected biomarkers by calculating the difference of the relative quantification of the biomarker and the normalization factor (formula 5). This normalization ensures comparability of test samples within a run (intra-run normalization).

$$N\,RQ_{jk} = RQ_{jk} - NF_k$$

Formula 5: Normalization of relative quantification: N RQ; j: counter of genes; k: counter of samples

For a reproducible diagnostic assay an inter-run calibration is required. Inter-run calibrations are calculated in an analogous manner to normalization. Firstly, a calibration factor is determined, wherein the calibration factor is the arithmetic mean of the normalized relative quantifications of inter-run calibrator. For inter-run calibration a pool of three PCa tissue samples and a pool of three tissue samples of BPH patients was included for each run/test plate. For each gene, the average expression in these two calibrator samples was calculated (formula 6).

$$CF_{jl} = \frac{\sum_{m=1}^{c} N\,RQ_{jlm}}{c}$$

Formula 6: Calculation of calibration factor: CF; c: number of inter-run calibrators; m: counter of inter-run calibrators; j: counter of genes; I: counter of runs/test plates

At least one calibrator sample has to be validly detected. Subsequently the calibration factor is used to calibrate the normalized relative quantification of the selected biomarkers by calculating the difference of the normalized relative quantification of the biomarker and the calibration factor (formula 7). This calibration ensures comparability of test samples between separate runs (inter-run calibration).

$$CN\,RQ_{jkl} = N\,RQ_{jkl} - CF_{jl}$$

Formula 7: Calibration of normalized relative quantification: CN RQ; j: counter of genes; k: counter of samples; I: counter of runs/test plates

The person skilled in the art is aware of alternative mathematical approaches of calculating gene normalization and calibration, which are also included in the present invention.

Normalized and calibrated results are subsequently checked by applying the second QC filter. This second QC filter determines if the subjects' sample contains a minimal amount of prostate cells which is sufficient for detection of the selected biomarkers for prostate cancer. As with the first QC filter the person skilled in the art is aware on how to select and validate suitable reference genes. For the prostate specific reference genes the inventors considered and tested several possible candidates. Suitable reference genes can be selected but are not limited to the following group: ACPP, KLK2, KLK3 and RDH11 (table 4). In a preferred embodiment of the invention the reference genes for the second QC filter are selected from the group of prostate specific reference genes KLK2 and KLK3.

For a sample to pass the second QC filter the tested reference genes have to show expression levels above the threshold level. The inventors determined the following threshold levels: for KLK2 a sample passes the threshold if the expression level is at least smaller than a calibrated relative quantification of 9.6676667 and for KLK3 the threshold is if the expression level is at least smaller than a calibrated relative quantification of 11.113. If a sample does not pass the second QC filter the sample is invalid and excluded from further analysis.

The calibrated relative quantifications of the selected biomarkers are then combined into a mathematical predictive model for diagnosis of the patient. The inventors found two applicable and predictive models using different sets of biomarkers. These two models were surprisingly found to achieve superior diagnostic predictive power than diagnostic methods of the state of the art (table 1). The identified models share two common biomarkers, which are essential to both models. The common biomarkers are PCaD4 and PCaD50. Hence in one aspect of the invention the method of diagnosis of PCa comprises the detection of at least biomarkers PCaD4 and PCaD50. The determined expression levels are then used within a mathematical model to obtain the patient's diagnosis.

Alternatively, the inventors identified a first model for diagnosis. This model combines the calibrated and normalized relative quantifications of the following biomarkers: PCaD4, PCaD54, PCaD50 and PCaD42. This model was found to have an AUC of 0.817 and a specificity at 90% sensitivity 0.512. A logistic regression model was fitted to the observed data for each transcript or gene to receive regression coefficients.

Alternatively, the inventors identified a second model for diagnosis. This model combines the calibrated and normalized relative quantifications of the following biomarkers: PCaD4, PCaD11, PCaD50, PCaD52, PCaD58 and PCaD2. This model was found to have an AUC of 0.829 and a specificity at 90% sensitivity 0.605. A logistic regression model was fitted to the observed data for each transcript or gene to receive regression coefficients. The threshold value is the minimal score value calculated from the calibrated and normalized relative expression of the selected biomarkers between the test sample and the control sample according to the applied model, at which score value the respective sample is designated as cancer-positive.

The logistic regression model allows the prediction of the tumour status of a test sample by combining the measured expression values of the sample with the determined regression coefficients of the model. For an estimated tumour probability of ≥50% a sample is classified as tumour positive for the respective model (model 1 or model 2). The invention is concerned with the quantification of the expression level of RNA biomarkers. After amplification, quantification is straightforward and can be accomplished by a number of methods. In the case when primers are used wherein at least one primer has a fluorescent dye attached, quantification is possible using the fluorescent signal from the dye. Various primer systems and dyes are available, such as SYBR green, Multiplex probes, TaqMan probes, molecular beacons and Scorpion primers. These are suitable for instance to carry out PCR-based methods such as quantitative reverse transcription PCR (qRT-PCR). Other possible means of quantification are for example northern blotting, next generation sequencing or absorbance measurements at 260 and 280 nm.

Any suitable method for the quantification of nucleic acids may be used to analyse the expression levels of the nucleic acids. In one embodiment of the invention, the analysis in the method is performed by a fluorescence based assay. In a preferred embodiment, the analysis is done by measuring the fluorescence of a labelled primer, labelled probe or a fluorescent detection agent (such as SYBR green). More preferably, this analysis of the expression level is performed by RT-qPCR. In this method, after reverse transcription, the sample is mixed with a forward and a reverse primer specific for at least one nucleic acid selected from the group of SEQ ID NO: 1 to 131, preferably selected from the group of primers and probes as listed in table 2 (SEQ ID NO: 136-174), followed by amplification. Probes or primers are designed such that they hybridize under stringent conditions to their respective target sequence.

In one embodiment, the analysis of the expression level is performed by next generation sequencing.

The invention also relates to a nucleic acid that hybridizes under stringent conditions to one of the nucleic acids according to SEQ ID NO: 1 to 131, or any part thereof, wherein said nucleic acid is a primer or a probe, preferably a labelled probe.

In a preferred embodiment of the invention, the nucleic acid that hybridizes under stringent conditions to one of the nucleic acids according to SEQ ID NO: 1-131 is about 5 to 500 nt in length, more preferably, 10 to 200 nt, even more preferably 10 to 100 nt. In the most preferred embodiment, said nucleic acid is 5, 6, 7, 8, 9, 10, 11, 12, 13, 14, 15, 16, 17, 18, 19, 20, 21, 22, 23, 24, 25, 26, 27, 28, 29 or 30 nt in length.

In one embodiment of the invention, the nucleic acid that hybridizes under stringent conditions to one of the nucleic acids according to SEQ ID NO: 1-131 comprises a detectable label. In an even more preferred embodiment, the nucleic acid that hybridizes under stringent conditions to one of the nucleic acids according to SEQ ID NO: 1-131 additionally comprises a quencher moiety.

The invention also relates to the use of a nucleic acid that hybridizes under stringent conditions to one of the nucleic acids according to SEQ ID NO: 1-131 for the diagnosis of prostate cancer.

The invention further relates to a nucleic acid with a sequence from the group of SEQ ID NO: 1 to 131, or the reverse complement thereof or any part thereof, or a nucleic acid that shares preferably at least 85%, 90%, 95% or 99% sequence identity with a nucleic acid according to any one of the nucleic acids according to SEQ ID NO: 1 to 131 or the reverse complement thereof or any part thereof, preferably the nucleic acid is selected from the group comprising SEQ ID No: 136-174.

The invention further relates to the use of a nucleic acid with a sequence from the group of SEQ ID NO: 1 to 131 for the diagnosis of prostate cancer.

The invention also relates to a kit for the diagnosis of prostate cancer comprising at least one nucleic acid that hybridizes under stringent conditions to one of the nucleic acids according to SEQ ID NO: 1-131. The kit may contain more than one nucleic acid. In a preferred embodiment, the kit additionally comprises reagents for nucleic acid amplification and/or quantification and/or detection. In another embodiment, the kit comprises control samples.

In an alternative embodiment, the invention relates to a method for the treatment and diagnosis of prostate cancer comprising the steps of analysing the expression level of the nucleic acid according to

SEQ ID NO: 1-3 and 19-50 in a sample from a patient, wherein, if the expression level of said nucleic acid is above a threshold value, the sample is designated as prostate cancer positive; and administering to the patient one or more Prostate Cancer Therapeutic Agents.

In one embodiment, the Prostate Cancer Therapeutic Agents comprises: Docetaxel (Taxotere®); Cabazitaxel (Jevtana®); Mitoxantrone (Novantrone®); Estramustine (Emcyt®); Doxorubicin (Adriamycin®); Etoposide (VP-16); Vinblastine (Velban®); Paclitaxel (Taxol®); Carboplatin (Paraplatin®); Abiraterone acetate, Bicalutamide, Casodex, Degarelix, Enzalutamide, Goserelin acetate, Leuprolide acetate, Prednisone, Sipuleucel-T, Radium 223 dichloride and/or Vinorelbine (Navelbine®).

One aspect of the present invention is a method for the ex-vivo diagnosis of prostate cancer comprising the steps of

> a) providing a sample from a patient suspected of having prostate cancer,
> b) analysing the expression level of at least the nucleic acids according to SEQ ID NO: 2 and SEQ ID NO: 20 in the sample,
> wherein, if the expression level of said nucleic acids is above a threshold value, the sample is designated as prostate cancer positive.

In another alternative aspect the present invention involves a method for the ex-vivo diagnosis of prostate cancer comprising the steps of

> a) providing a sample from a patient suspected of having prostate cancer,
> b) analysing the expression level of at least the nucleic acids according to SEQ ID NO: 2, SEQ ID NO: 19-20 and SEQ ID NO: 35-37 in the sample,

wherein, if the expression level of said nucleic acids is above a threshold value, the sample is designated as prostate cancer positive.

In another alternative aspect the present invention involves a method for the ex-vivo diagnosis of prostate cancer comprising the steps of

a) providing a sample from a patient suspected of having prostate cancer,
b) analysing the expression level of at least the nucleic acids according to SEQ ID NO: 1-3, SEQ ID NO: 20-34 and SEQ ID NO: 38-50 in the sample,

wherein, if the expression level of said nucleic acids is above a threshold value, the sample is designated as prostate cancer positive.

In one embodiment of the present invention the sample for the method of the invention is selected from the group comprising prostate tissue, biopsy material, lymph nodes, urine, ejaculate, blood, blood serum, blood plasma, circulating tumour cells in blood or lymph, any tissue suspected of containing metastases as well as any source that may contain prostate tumour cells or parts thereof, including vesicles like exosomes, micro vesicles, and others as well as free or protein-bound RNA molecules derived from prostate tumour cells.

In a preferred embodiment of the invention the sample is a urine sample, in a more preferred embodiment the sample is urinary sediment. In an even more preferred embodiment the sample is a urine sample obtained after digital rectal examination (DRE): DRE-urine, in a most preferred embodiment the sample is urinary sediment obtained after DRE: DRE urinary sediment. DRE is a routinely performed examination method which allows getting urine samples that contain a certain amount of prostate cells. Obtained DRE urine samples are centrifuged and washed two times using PBS to isolate cells. The isolated cells are subsequently lysed and the contained RNA is isolated according to commonly known and well-established techniques. The skilled person in the art is well aware of different applicable approaches of obtaining purified RNA from cellular samples.

In another embodiment of the invention the method involves analysis of the expression level by measuring the fluorescence of a labelled primer, labelled probe or a fluorescent detection agent.

In another embodiment of the invention the method involves analysis of the expression level by qRT-PCR.

In another embodiment of the invention the method involves normalization of the expression levels of biomarkers to general and prostate cell specific reference genes.

In a preferred embodiment of the invention the general and prostate cell specific reference genes are selected from the group comprising: EMC7, RAB7A, VCP, KLK2 and KLK3.

In another embodiment of the method of the invention the method involves using general and prostate cell specific reference genes to evaluate sample quality. The expression levels of the general and prostate cell specific reference genes are used to provide at least one quality control filter, only samples which pass the at least one quality control filter are included in analysis.

In another aspect of the invention, the invention involves nucleic acids that hybridize under stringent conditions to one of the nucleic acids of SEQ ID NO: 1-3 and 19-50. This means that the invention involves nucleic acids that share at least 85%, 90%, 91%, 92%, 93%, 94%, 95%, 96%, 97 %, 98% or 99% sequences identity with one of the sequences of SEQ ID NO: 1-3 and 19-50 or the reverse complement thereof or any partial sequence thereof. Preferably the nucleic acid is selected from the group comprising SEQ ID No: 136-144 and 148-162, wherein these nucleic acids can be used primers or probes to detect the sequences of SEQ ID NO: 1-3 and 19-50 or sequences that share at least 85%, 90%, 91%, 92%, 93%, 94%, 95%, 96%, 97 %, 98% or 99% sequences identity thereof.

The above nucleic acids which can be used as primer or probes are about 10 to 100 nucleotides in length, more preferably about 10-50 nucleotides, even more preferably about 15-40 nucleotides, even more preferably about 15-35 nucleotides and most preferably about 15-25 nucleotides.

The above nucleic acids may further comprise a detectable label. In a preferred embodiment the detectable label is a fluorescent label which is covalently attached to the nucleic acid. The fluorescent label may be further combined with at least one quencher molecule which is also covalently attached to the nucleic acid and which may quench the fluorescence signal of the fluorescent label.

The above described nucleic acid can be used for the diagnosis of prostate cancer.

A nucleic acid with one of the sequences of SEQ ID NO: 1-131, or the reverse complement thereof or any part thereof, or a nucleic acid that shares preferably at least 85%, 90%, 91%, 92%, 93%, 94%, 95%, 96%, 97 %, 98% or 99% sequence identity with one of the sequences of SEQ ID NO: 1-131, or the reverse complement thereof or any part thereof, preferably the nucleic acid is selected from the group comprising SEQ ID No: 136-174 wherein these nucleic acids can be used primers or probes to detect the sequences of SEQ ID NO: 1-131 or sequences that share at least 85%, 90%, 91%, 92%, 93%, 94%, 95%, 96%, 97 %, 98% or 99% sequences identity thereof.

The above nucleic acids may further comprise a detectable label. In a preferred embodiment the detectable label is a fluorescent label which is covalently attached to the nucleic acid. The fluorescent label may be further combined with at least one quencher molecule which is also covalently attached to the nucleic acid and which may quench the fluorescence signal of the fluorescent label. The describes labelled primers or probes can be used for the diagnosis of prostate cancer.

In another aspect of the invention, the invention also involves a kit for the diagnosis of prostate cancer comprising any of the above described nucleic acid according to any one of SEQ ID NO: 1-3 and 19-50, or SEQ ID NO: 1-131, or SEQ ID NO: 136-144 and 148-162, or SEQ ID NO: 136-174 and optionally also comprising reagents for nucleic acid amplification and/or quantification and/or detection.

In a further aspect of the invention, the invention also involves a method for the treatment and diagnosis of prostate cancer comprising the steps of

a) analysing the expression level of the nucleic acid according to any one of SEQ ID NO: 2 and 20, or SEQ ID NO: 2, 19-20 and 35-37, or SEQ ID NO: 1-3, 20-34 and 38-50 in a sample from a patient,
b) wherein, if the expression level of said nucleic acid is above a threshold value, the sample is designated as prostate cancer positive; and
c) administering to the patient one or more Prostate Cancer Therapeutic Agents.

Possible known Prostate Cancer Therapeutic Agents are selected but not limited to the group comprising: Docetaxel (Taxotere®); Cabazitaxel (Jevtana®); Mitoxantrone (Novantrone®); Estramustine (Emcyt®); Doxorubicin (Adriamycin®); Etoposide (VP-16); Vinblastine (Velban®); Paclitaxel (Taxol®); Carboplatin (Paraplatin®); Abiraterone acetate, Bicalutamide, Casodex, Degarelix, Enzalutamide, Goserelin acetate, Leuprolide acetate, Prednisone, Sipuleucel-T, Radium 223 dichloride and/or Vinorelbine (Navelbine®).

In a further aspect of the invention, the invention involves a method for the diagnosis of prostate cancer comprising the steps of

a) analysing the expression level of at least the nucleic acids according to SEQ ID NO: 2 and 20, or SEQ ID NO: 2, 19-20 and 35-37, or SEQ ID NO: 1-3, 20-34 and 38-50 in a sample from a patient,
b) wherein, if the expression level of said nucleic acids is above a threshold value, the sample is designated as prostate cancer positive.

The below examples present in detail the methods applied for identifying and evaluating novel biomarkers and reference genes for diagnosis of PCa. The newly identified biomarkers and the above describes diagnostic methods based on these biomarkers allow a more accurate and sensitive diagnosis of the disease than current biomarkers.

**Citations**

Aubin SMJ et al. J Urol. 2010;184:1947-52.

Yoav Benjamini and Yosef Hochberg. Controlling false discovery rate: a practical and powerful approach to multiple testing: Journal of the Royal Statistical Society 1995.

Cucchiara, V., Cooperberg, M. R., Dall'Era, M., Lin, D. W., Montorsi, F., Schalken, J. A. u. Evans, C. P.: Genomic Markers in Prostate Cancer Decision Making. European urology (2017)

Duffy, Michael J. "Biomarkers for prostate cancer: prostate-specific antigen and beyond" Clinical Chemistry and Laboratory Medicine (CCLM), (2020)

Gleason, D. F. (1977). "The Veteran's Administration Cooperative Urologic Research Group: histologic grading and clinical staging of prostatic carcinoma". In Tannenbaum, M. Urologic Pathology: The Prostate. Philadelphia: Lea and Febiger. pp. 171-198.

Guescini M, Sisti D, Rocchi MB, Stocchi L, Stocchi V. A new real-time PCR method to overcome significant quantitative inaccuracy due to slight amplification inhibition. BMC Bioinformatics. 2008 Jul 30;9:326. doi: 10.1186/1471-2105-9-326

Haese A et al. Eur Urol. 2008;54:1081-8.

Irshad S et al. Sci Transl Med 2013;5:202ra122.

Jansen FH et al. Eur Urol. 2009;55:563-74.

Jansen FH, van Schaik RH, Kurstjens J, Horninger W, Klocker H, Bektic J, et al. Prostate-specific antigen (PSA) isoform p2PSA in combination with total PSA and free PSA improves diagnostic accuracy in prostate cancer detection. Eur Urol 2010;57:921-7.

Lendinez-Cano G. Prospective study of diagnostic accuracy in the detection of high-grade prostate cancer in biopsy-naive patients with clinical suspicion of prostate cancer who underwent the Select MDx test. Prostate. 2021 Sep;81(12):857-865

Moyer VA et al. Ann Intern Med. 2012;157:120-134.

Salagierski M et al. J Urol 2012;187: 795-801.

Thompson IM et al. J Am Med Assoc. 2005;294:66-70.

Van Gils MP et al. Clin Cancer Res. 2007;13:939-43.

Vickers AJ, Cronin AM, Aus G, Pihl CG, Becker C, Pettersson K, et al. A panel of kallikrein markers can reduce unnecessary biopsy for prostate cancer: data from the European Randomized Study of Prostate Cancer Screening in Goteborg, Sweden. BMC Med 2008;6:19.

**Examples**

Materials and methods

Clinical cohort

Prostate carcinoma (PCa) patients who underwent radical prostatectomy (RPE) or surgery to remove a benign prostate hyperplasia (BPH) were included in a retrospective clinical cohort aiming at identifying novel biomarkers for PCa. Approval from the local ethics committee as well as informed consent from the patients were obtained according to the legal regulations. Data on the clinical follow-up were collected for at least five years for the PCa patients.

Prostate tissue samples from a cohort of 40 PCa patients and 8 BPH patients were used for identification of diagnostically relevant biomarkers by genome-wide RNA sequencing. Four PCa groups were defined based on staging according to Gleason (The Veteran's Administration Cooperative Urologic Research Group: histologic grading and clinical staging of prostatic carcinoma; in Tannenbaum, M. Urologic Pathology: The Prostate, Philadelphia: Lea and Febiger. Pp. 171-198) as well as the presence of metastases in the adjacent lymph nodes upon RPE (see Table 5). The composition of test cohorts is also shown in figure 1.

Selected biomarker candidates were further validated by custom microarrays and quantitative reverse-transcription real-time PCR (qRT-PCR) on cohorts comprising 203 (39 control BPH, 164 tumour samples) and 338 patients (126 control BPH samples, 212 tumour samples), respectively.

Table 5: PCa cohort for genome-wide RNA sequencing screening: The control group (C) consists of BPH samples. The very low risk (V) and low risk (L) groups comprise samples from patients graded with Gleason Score (GS)<7 and =7, respectively, all without lymph node metastases (pN0). The medium risk (M) group comprises cases with GS<=7 and exhibiting lymph node metastases (pN+); and the high risk (H) group consist of tissues with GS>7. For the latter, pairs of tumour and tumour-free tissue samples obtained from the same patient were analysed.

| Group | C | V | L | M | H | | | |
|---|---|---|---|---|---|---|---|---|
| Gleason score | BPH | GS<7 | GS=7 | GS<=7 | GS>7 | | | |
| lymph node metastasis | - | pN0 | pN0 | pN+ | pN0 | | pN+ | |
| tissue | control | tumour | tumour | tumour | tumour | tumour-free | tumour | tumour-free |

(continued)

| Group | C | V | L | M | H | | | |
|---|---|---|---|---|---|---|---|---|
| number of samples | 8 | 8 | 8 | 8 | 8 | 8 | 8 | 8 |

Prostate tissue samples

Prostate tissue samples were obtained from surgery and stored in liquid nitrogen. Prostate tissue samples obtained from radical prostatectomies (RPEs) of prostate carcinoma (PCa) patients were divided into tumour and tumour-free samples. Prostate tissue samples from patients with benign prostate hyperplasia (BPH) were used as controls. Patient consent was always given.

To verify the status of the samples and their tumour cell content, all samples were divided into series of cryosections. To this end, frozen tissue samples were embedded in Tissue-Tek OCT-compound (Sakura Finetek GmbH) and fixed on metal indenters by freezing. Cryosections were prepared using a cryomicrotome (Leica) equipped with a microtome blade C35 (FEATHER) cooled to -28°C. Every sample was cut into a total of 208 cryosections, 4 of which were HE-stained and evaluated by a pathologist with respect to their tumour cell content. This yielded 3 stacks of consecutive cryosections, each of which was flanked by HE-stained sections. Only stacks that were flanked on either side by sections containing at least 60% or at most 5% tumour cells were used as tumour or tumour-free samples, respectively. 50 cryosections of the stacks chosen were then subjected to RNA preparation.

RNA isolation

Total RNA was isolated from cryo-preserved tissue using Qiazol and the miRNeasy Mini Kit on the QIAcube (all from Qiagen) with manual subsequent DNase I digestion. RNA concentration was determined using a Nanodrop 1000 (Peqlab). RNA integrity was verified on an Agilent Bioanalyzer 2100 (Agilent Technologies, Palo Alto, CA), and only RNA samples with an RNA-Integrity-Number (RIN) of at least 6 were further processed.

Genome-wide long-RNA next generation sequencing

Genome-wide long RNA sequencing was performed using a subset of the retrospective PCa cohort comprising 8 prostate tissue samples from benign prostate hyperplasia (BPH) as a control and 56 samples from patients with prostate cancer (including tumour and tumour-free tissue pairs from samples with Gleason score >7). 1 $\mu$g of total RNA was depleted of ribosomal RNA using the Ribo-Zero rRNA Removal Kit (Epicentre). Sequencing libraries were prepared from 50 ng of rRNA-depleted RNA using ScriptSeq v2 RNA-Seq Library Preparation Kit (Epicentre). The di-tagged cDNA was purified using the Agencourt AMPure XP System Kit (Beckman Coulter). PCR was carried out through 10 cycles to incorporate index barcodes for sample multiplexing and amplify the cDNA libraries. The quality and concentration of the amplified libraries were determined using a DNA High Sensitivity Kit on an Agilent Bioanalyzer (Agilent Technologies). 4 ng each of 8 samples were pooled and size-selected on 2% agarose gels using agarose gel electrophoresis. The sample range between 150 bp and 600 bp was gel-excised and purified with the MinElute Gel Extraction Kit (Qiagen), according to manufacturer's instructions. The purified libraries were quantified on an Agilent Bioanalyzer using a DNA High Sensitivity Chip (Agilent Technologies). Every purified and size-selected library pool was then loaded onto an Illumina HiSeq2000 flow cell, distributing it among all lanes. Cluster generation was performed using TruSeq PE Cluster Kits v3 (Illumina Inc.) in an Illumina cBOT instrument following the manufacturer's protocol. Sequencing was performed on an Illumina HiSeq2000 sequencing machine (Illumina, Inc.). The details of the sequencing runs were as follows: paired-end sequencing strategy; 101 cycles for Read1, 7 cycles for index sequences, and 101 cycles for Read2.

Analysis of sequencing data: Raw data preparation

Raw sequencing data comprising base call files (BCL files) was processed with CASAVA v1.8.1 (Illumina) resulting in FASTQ files. FASTQ files contain for each clinical sample all sequenced RNA fragments, in the following referred to as "reads". Specific adapter sequences were removed by using cutadapt v1.6 (http://code.google.com/p/cutadapt/).

Analysis of sequencing data: Genome mapping and transcript assembling:

Reads were mapped to the human genome (assembly GRCh37/hg19) using segemehl v0.1.4-382 and TopHat v2.0.87. Novel transcripts, i.e. transcripts not annotated in Gencode v17, were assembled using Cufflinks v2.1.18 and Cuffmerge v2.1.1. All novel transcripts and all known Gencode v17 transcripts were combined into a comprehensive annotation set.

Analysis of sequencing data: Statistical analysis

Htseq-count v0.6.0 (http://www-huber.embl.de/users/anders/HTSeq/doc/count.html) was used to compute the read counts per transcript and gene that are contained in the comprehensive annotation set of novel and known transcripts. Differentially expressed transcripts and genes were identified using R and the Bioconductor library DeSeq2 v1.81. Raw read counts were normalized, and variance stabilized (vst transformation). A negative binomial log-linear model was fitted to the read counts for each transcript or gene, and coefficients distinct from zero identified by a likelihood ratio test. False discovery rate was controlled by Benjanimi-Hochberg adjustment.

Validation by custom microarrays

Based on the sequencing results custom microarrays with 180,000 probes (Agilent SurePrint G3 Custom Exon Array, 4x180K, Design-ID 058029) were designed comprising mRNAs, long noncoding RNAs (gencode v15), new transcripts and all transcripts found by RNA sequencing to be expressed differentially between tumour and control tissue samples. Probe design was done using the Agilent custom design tool eArray.

The microarray screening was performed using the retrospective PCa cohort comprising 40 prostate tissue samples and 8 samples from patients with benign prostate hyperplasia (BPH) as a control as well as 164 and 52 tumour and tumour-free tissue samples, respectively, of prostate cancer patients after radical prostatectomy. Using the Quick Amp Labeling Kit (Agilent) cRNA was synthesized from 200 ng total RNA, and 1650 ng cRNA were hybridized on the arrays (Agilent Gene Expression Hybridization Kit).

Analysis of RNA custom microarray data:

Differential expression analysis in the validation cohort was only conducted for patients not included in the exploration cohort (164 PCa patients and 39 BPH patients). Differentially expressed probes were identified by using R v3.2.2 and the Bioconductor package limma v3.24.15. Quality control of arrays was performed by checking distribution of "bright corner", "dark corner" probes, and relative spike-in concentration versus normalized signal. To retrieve a set of probes mapping to unique genomic positions in hg19 BLAT v35 with the parameter -minIdentity = 93 was used. All probes mapping to more than one distinct genomic region were discarded. Normalization between arrays was done by using quantile normalization. In order to reduce the number of tests non-specific filtering was applied as follows: The expression of a probe must be larger than background expression in at least 20 arrays. Background expression is defined by the mean intensity plus three times the standard deviation of negative control spots (Agilent's 3xSLv spots). In addition, a probe must exhibit a nonspecific change of expression of at least IQR greater than 0.5. Finally, a linear model was fitted using the R package limma and reliable variance estimates were obtained by Empirical Bayes moderated t-statistics. False discovery rate was controlled by Benjamini-Hochberg adjustment.

Validation by quantitative real-time PCR

For validation of the results obtained by next generation sequencing and microarray screening were analysed using quantitative real-time PCR. cDNA was synthesized from 100 ng total RNA using the High-Capacity Reverse transcription kit (Applied Biosystems) and random primers according to manufacturer's instructions. Subsequent PCR assays were run using 4 $\mu$l of the diluted cDNA. Quantitative real-time PCR was performed using custom- and pre-designed TaqMan Gene Expression Assays (Applied Biosystems) for reference and biomarker transcripts on an Applied Biosystems 7900HT Real-Time PCR System. The tested biomarkers and reference genes are listed in table 2 and the used primer and probes are listed in table 3.

All samples were measured in triplicate and the means of these measurements were used for further calculations. All samples were checked for sufficient quality using the above described QC filters. Only samples that passed QC filters were valid and suitable to be included into the final analysis. The first QC filter tests for sufficient amount of cells in the sample, while the second QC filter confirms that the amount of prostate cells in the sample is sufficient. This was further combined with evaluation of amplification quality, to ensure that replicate measurements of a sample are reproducible and consistent and that the amplification satisfies the minimal standards of the MIQE guidelines.

Validation in DRE urine samples: DRE urine sample collection and RNA isolation

Urine samples were collected after digital rectal examination (DRE) of the prostate (DRE urine). This routinely performed examination method allows getting urine samples that contain a certain amount of prostate cells. The DRE urine samples were centrifuged and washed two times using PBS. The resulting cell pellet was resuspended in 700μl Qiazol. Total RNA was isolated using the miRNeasy Mini Kit on the QIAcube (all from Qiagen) with manual subsequent DNase I digestion. RNA concentration was determined using a Nanodrop 1000 (Peqlab). RNA integrity was verified on an Agilent Bioanalyzer 2100 (Agilent Technologies, Palo Alto, CA).

Quantitative real-time PCR screening of DRE urine samples

cDNA was synthesized from $2 \times 50$ ng total RNA using the Superscript III Reverse transcriptase (Applied Biosystems) and random primers according to manufacturer's instructions. Subsequent PCR assays were run using 4 μl of cDNA. Quantitative real-time PCR was performed using custom and pre-designed TaqMan Gene Expression Assays (Applied Biosystems) for housekeeping (PSA) and target transcripts on an Applied Biosystems 7900HT Real-Time PCR System. All samples were measured in duplicate and the means of these measurements were used for further calculations.

Genome-wide long-RNA next generation sequencing of DRE urine samples

For genome-wide long RNA sequencing total RNA from 11 DRE urine samples was precipitated using ethanol to concentrate the RNA amount and resuspended in 10 μl RNase free water. The rRNA removal was performed with 4 ng of total RNA using the Low input Ribo-Zero rRNA Removal Kit (Epicentre, modified by Clontech), resulting in 10 μl rRNA depleted RNA. Sequencing libraries were prepared from 8 μl rRNA-depleted RNA using the SMARTER stranded RNAseq Kit (Clontech). The di-tagged cDNA was purified using the Agencourt AMPure XP System Kit (Beckman Coulter). PCR was carried out through 18 cycles to incorporate index barcodes for sample multiplexing and amplify the cDNA libraries. The quality and concentration of the amplified libraries were determined using a DNA High Sensitivity Kit on an Agilent Bioanalyzer (Agilent Technologies). Samples were pooled and cluster generation was performed using 15 pmol/l of the pooled library and the TruSeq PE Cluster Kit v4 (Illumina Inc.) in an Illumina cBOT instrument following the manufacturer's protocol. Sequencing was performed using the HiSeq SBS v4 sequencing reagents (250 cycles) on an Illumina HiSeq2500 sequencing machine (Illumina, Inc.). The details of the sequencing run were as follows: paired-end sequencing strategy; 126 cycles for Read1, 7 cycles for index sequences, and 126 cycles for Read2.

Statistical analysis of the qRT-PCR results from DRE urines

For relative quantification, changes in gene expression of each sample were analysed relative to the average expression of the gene within the same run. Data normalization was carried out against the general reference genes as listed table 4: EMC7 (SEQ ID NO: 74-77), RAB7A (SEQ ID NO: 78-95) and VCP (for SEQ ID NO: 96-131). Measurements were calibrated between different runs by calibrator samples from pooled positive control samples of patients with diagnosed benign prostate hyperplasia (BPH) or with diagnosed prostate cancer.

After normalization and calibration, relative expression levels of the biomarkers were compared between tumour and control samples employing Mann-Whitney U tests. Receiver-operating characteristic (ROC) curves, representing a measure of diagnostic power of each marker by the area under the curve (AUC), were calculated using the package pROC. Variable selection was done by variable importance by random forest and stepwise model selection (logistic regression: top-down by AIC). Final model were trained by logistic regression and ridge logistic regression. All statistical analyses were carried out using R statistical software.

Results

The transcriptomes of 40 PCa tumour samples and 16 tumour-free samples obtained upon RPE and 8 BPH prostate tissue samples as benign, non-tumour controls were analysed using strand-specific, paired-end long RNA next generation sequencing (NGS). Approximately 150 cryosections per sample in at least three segments were prepared, aiming at an optimal data quality and robustness of the analysis. Upon pathological evaluation, only segments satisfying a maximal and minimal tumour cell count of 60% and 5% in tumour and tumour free samples, respectively, were retained for further analysis. The transcriptome sequencing (RNAseq) approach aimed at a comprehensive identification and quantification of RNAs expressed in normal or cancer prostate tissue. All classes of coding and long non-coding transcripts independent of polyadenylation status were sequenced. Large input masses of RNA were used to ensure high library complexity. Furthermore, on average 200 M paired-end reads $2 \times 100$ nt per library were sequenced, enabling the assembly of novel lowly expressed transcripts due to high coverage. This approach outperformed most comparable published studies that analysed larger numbers of samples. In total, approx. 3000 novel transcripts that did not show an exonic overlap with transcripts annotated in Gencode v17 were assembled. At a false discovery rate of 0.01, 6442 differentially expressed genes across all contrasts were observed.

The results successfully reproduced the majority of transcripts previously reported to be differentially expressed between prostate tumour and normal tissue. In addition, a number of novel PCa-associated transcripts were identified, which can be used to develop assays for the diagnosis of PCa. The most promising transcripts were selected for validation in a test cohort of PCA tumour and BPH control samples by qRT-PCR.

Several of these novel biomarker candidates significantly surpass the specificity and sensitivity of the biomarker PCA3, which is already used for PCa diagnosis. In the sequencing cohort, PCA3 proved to be clearly associated with PCa, yet with a strong tendency to a decline in the high-risk group (Fig. 2).

The experimental results demonstrate high specificity and sensitivity of the novel biomarkers for the detection of PCa. As shown in figure 3 which compares the differences in expression levels of various PCa biomarkers between PCa samples and BPH control samples, results for both the newly identified biomarkers (ProstaCheck) and previously established biomarkers (SelectMDx and PCA3) are shown. Figure 3 clearly indicates that a more pronounced difference in expression levels between PCa samples and control samples could be observed for the newly identified biomarkers as opposed to the previously known biomarkers. Therefore, assays can be set up based on the measurement of these newly discovered biomarkers alone or in combination (or in combination with other markers) in all sources that may contain prostate tumour cells or parts thereof (including vesicles like exosomes, microvesicles, and others as well as free or protein-bound RNA molecules deriving from prostate tumour cells) to be used for the diagnosis of PCa. As shown in table 1 these new assay provide superior diagnostic reliability.

The advantages of a diagnostic assays based on these biomarkers allows a dramatically lower false-positive rate compared to current assays and measuring their expression levels in urine sample avoid having to perform unnecessary invasive prostate biopsies.

**Brief description of the Figures**

Figure 1: Processed cohorts for the development of ProstaCheck

Figure 2: Box plot of RNA-seq data for transcript PCA3. Results from RNA sequencing of the retrospective PCa cohort comprising 8 prostate tissue samples from benign prostate hyperplasia as a control (C), 8 PCa tumour samples each of groups V (very low risk; Gleason score <7, pN0), L (low risk; Gleason score =7, pN0), and M (medium risk; Gleason score <=7, pN+), as well as 16 pairs of tumour and tumour-free tissue samples from group H (high risk; Gleason score >7).

Figure 3: Expression in transcripts per million (TPM) of known diagnostic markers (PCA3, HOXC6, DLX1) compared to genes of the ProstaCheck test in urinary sediment (RNA-seq of 6 BPH and 5 PCa urinary sediments). Genes included in ProstaCheck depict on average a higher expression in urinary sediments than known markers.

**Sequence listing**

The sequences of the sequence listing are filed separately. Below table 6 discloses the Ensembl transcript IDs or Ensembl gene IDs, when no known transcript variant was available, which are represented by the individual SEQ ID NOs.

Table 6: Association of SEQ ID NOs of the invention and Ensembl transcript variant/gene IDs

| SEQ ID NO | Biomarker | Gene-symbol | Ensembl transcript variant/gene IDs (Gencode V41) |
|---|---|---|---|
| 1 | PCaD2 | CPNE4 | ENSG00000196353: chromosome: GRCh38:3: 131533555: 132285410:-1 DNA |
| 2 | PCaD4 | | |
| 3 | PCaD11 | TAPIR | ENST00000366424.2 |

(continued)

| SEQ ID NO | Biomarker | Gene-symbol | Ensembl transcript variant/gene IDs (Gencode V41) |
|---|---|---|---|
| 4 | PCaD32 | KLK3 | ENST00000601503.5 |
| 5 | | | ENST00000596185.5 |
| 6 | | | ENST00000326003.7 |
| 7 | | | ENST00000422986.6 |
| 8 | | | ENST00000601349.5 |
| 9 | | | ENST00000595151.5 |
| 10 | | | ENST00000597286.5 |
| 11 | | | ENST00000596333.1 |
| 12 | | | ENST00000597483.5 |
| 13 | | | ENST00000593997.5 |
| 14 | | | ENST00000595392.5 |
| 15 | | | ENST00000595952.5 |
| 16 | | | ENST00000360617.7 |
| 17 | | | ENST00000598145.1 |
| 18 | | | ENST00000601812.1 |
| 19 | PCaD42 | HOXB13 | ENST00000290295.8 |
| 20 | PCaD50 | PCAT14 | ENSG00000280623: chromosome: GRCh38:22: 23536881: 23547797:1 DNA |
| 21 | PCaD52 | CPNE4 | ENST00000514439.1 |
| 22 | | | ENST00000617767.5 |
| 23 | | | ENST00000512055.5 |
| 24 | | | ENST00000429747.6 |
| 25 | | | ENST00000515418.1 |
| 26 | | | ENST00000503204.1 |
| 27 | | | ENST00000512332.5 |
| 28 | | | ENST00000511604.5 |
| 29 | | | ENST00000502818.5 |
| 30 | | | ENST00000505881.5 |
| 31 | | | ENST00000514999.5 |
| 32 | | | ENST00000505957.1 |
| 33 | | | ENST00000506687.5 |
| 34 | | | ENST00000505331.1 |
| 35 | PCaD54 | MSMB | ENST00000581478.5 |
| 36 | | | ENST00000582163.3 |
| 37 | | | ENST00000663171.1 |

(continued)

| SEQ ID NO | Biomarker | Gene-symbol | Ensembl transcript variant/gene IDs (Gencode V41) |
|---|---|---|---|
| 38 | PCaD58 | FOLH1 | ENST00000256999.7 |
| 39 | | | ENST00000458311.6 |
| 40 | | | ENST00000356696.7 |
| 41 | | | ENST00000525826.5 |
| 42 | | | ENST00000340334.11 |
| 43 | | | ENST00000533034.1 |
| 44 | | | ENST00000532018.1 |
| 45 | | | ENST00000525629.1 |
| 46 | | | ENST00000526226.1 |
| 47 | | | ENST00000529646.5 |
| 48 | | | ENST00000533510.5 |
| 49 | | | ENST00000529117.1 |
| 50 | | | ENST00000529648.1 |
| 51 | PCaD91 | KLK2 | ENST00000593493.5 |
| 52 | | | ENST00000595375.5 |
| 53 | | | ENST00000597509.5 |
| 54 | | | ENST00000596950.5 |
| 55 | | | ENST00000600690.5 |
| 56 | | | ENST00000599280.5 |
| 57 | | | ENST00000594174.5 |
| 58 | | | ENST00000391810.6 |
| 59 | | | ENST00000597727.5 |
| 60 | | | ENST00000595316.5 |
| 61 | | | ENST00000599121.5 |
| 62 | | | ENST00000325321.8 |
| 63 | | | ENST00000600755.5 |
| 64 | | | ENST00000600866.1 |
| 65 | | | ENST00000358049.8 |
| 66 | | | ENST00000597439.1 |
| 67 | | | ENST00000601743.1 |
| 68 | | | ENST00000599568.1 |
| 69 | | | ENST00000595173.1 |
| 70 | | | ENST00000601114.1 |
| 71 | | | ENST00000595050.1 |
| 72 | | | ENST00000597911.1 |
| 73 | | | ENST00000597461.1 |

(continued)

| SEQ ID NO | Biomarker | Gene-symbol | Ensembl transcript variant/gene IDs (Gencode V41) |
|---|---|---|---|
| 74 | PCaD92 | EMC7 | ENST00000256545.9 |
| 75 | | | ENST00000527822.5 |
| 76 | | | ENST00000528949.1 |
| 77 | | | ENST00000532113.1 |
| 78 | PCaD93 | RAB7A | ENST00000676214.1 |
| 79 | | | ENST00000676425.1 |
| 80 | | | ENST00000675712.1 |
| 81 | | | ENST00000675864.1 |
| 82 | | | ENST00000490093.6 |
| 83 | | | ENST00000675342.1 |
| 84 | | | ENST00000493186.6 |
| 85 | | | ENST00000674748.1 |
| 86 | | | ENST00000265062.8 |
| 87 | | | ENST00000482525.5 |
| 88 | | | ENST00000491681.1 |
| 89 | | | ENST00000675497.1 |
| 90 | | | ENST00000674589.1 |
| 91 | | | ENST00000464496.5 |
| 92 | | | ENST00000483906.5 |
| 93 | | | ENST00000485280.1 |
| 94 | | | ENST00000676147.1 |
| 95 | | | ENST00000674593.1 |
| 96 | PCaD94 | VCP | ENST00000679902.1 |
| 97 | | | ENST00000678650.1 |
| 98 | | | ENST00000678018.1 |

(continued)

| SEQ ID NO | Biomarker | Gene-symbol | Ensembl transcript variant/gene IDs (Gencode V41) |
|---|---|---|---|
| 99 | | | ENST00000679800.1 |
| 100 | | | ENST00000677257.1 |
| 101 | | | ENST00000678465.1 |
| 102 | | | ENST00000681537.1 |
| 103 | | | ENST00000358901.11 |
| 104 | | | ENST00000448530.6 |
| 105 | | | ENST00000679647.1 |
| 106 | | | ENST00000681335.1 |
| 107 | | | ENST00000417448.2 |
| 108 | | | ENST00000680916.1 |
| 109 | | | ENST00000679599.1 |
| 110 | | | ENST00000679862.1 |
| 111 | | | ENST00000681690.1 |
| 112 | | | ENST00000679204.2 |
| 113 | | | ENST00000676836.2 |
| 114 | | | ENST00000493886.5 |
| 115 | | | ENST00000479300.2 |
| 116 | | | ENST00000466100.1 |
| 117 | | | ENST00000480327 .2 |
| 118 | | | ENST00000681125.1 |
| 119 | | | ENST00000680834.1 |
| 120 | | | ENST00000680520.1 |
| 121 | | | ENST00000681789.1 |
| 122 | | | ENST00000680731.1 |
| 123 | | | ENST00000681562.1 |
| 124 | | | ENST00000680079.1 |
| 125 | | | ENST00000679449.1 |
| 126 | | | E NST00000680900.1 |
| 127 | | | ENST00000681386.1 |
| 128 | | | ENST00000679901.1 |
| 129 | | | ENST00000679392.1 |
| 130 | | | ENST00000680108.1 |
| 131 | | | ENST00000680575.1 |
| 132 | | | ENST00000412654.1 |
| 133 | PCaD30 | PCA3 | ENST00000644302.1 |
| 134 | | | ENST00000645809.1 |
| 135 | | | ENST00000646854.1 |

(continued)

| SEQ ID NO | Biomarker | Gene-symbol | Ensembl transcript variant/gene IDs (Gencode V41) |
|---|---|---|---|
| 136-177 | Primer and probe sequences as disclosed in table 3 | | |

**Claims**

1. A method for the ex-vivo diagnosis of prostate cancer comprising the steps of

   a) providing a sample from a patient suspected of having prostate cancer,
   b) analysing the expression level of at least the nucleic acids according to SEQ ID NO: 2 and SEQ ID NO: 20 in the sample,
   wherein, if the expression level of said nucleic acids is above a threshold value, the sample is designated as prostate cancer positive.

2. A method for the ex-vivo diagnosis of prostate cancer comprising the steps of

   a) providing a sample from a patient suspected of having prostate cancer,
   b) analysing the expression level of at least the nucleic acids according to SEQ ID NO: 2, SEQ ID NO: 19-20 and SEQ ID NO: 35-37 in the sample,
   wherein, if the expression level of said nucleic acids is above a threshold value, the sample is designated as prostate cancer positive.

3. A method for the ex-vivo diagnosis of prostate cancer comprising the steps of

   a) providing a sample from a patient suspected of having prostate cancer,
   b) analysing the expression level of at least the nucleic acids according to SEQ ID NO: 1-3, SEQ ID NO: 20-34 and SEQ ID NO: 38-50 in the sample,
   wherein, if the expression level of said nucleic acids is above a threshold value, the sample is designated as prostate cancer positive.

4. The method according to any one of the preceding claims, wherein the sample is selected from the group comprising prostate tissue, biopsy material, lymph nodes, urine, ejaculate, blood, blood serum, blood plasma, circulating tumour cells in blood or lymph, any tissue suspected of containing metastases as well as any source that may contain prostate tumour cells or parts thereof, including vesicles like exosomes, micro vesicles, and others as well as free or protein-bound RNA molecules derived from prostate tumour cells.

5. The method according to any one of the preceding claims, wherein the analysis of the expression level is performed by measuring the fluorescence of a labelled primer, labelled probe or a fluorescent detection agent, preferably the analysis of the expression level is performed by qRT-PCR.

6. The method according to any one of the preceding claims, wherein the expression levels are normalized to general and prostate cell specific reference genes.

7. The method according to any one of the preceding claims, wherein the general and prostate cell specific reference genes are used to evaluate sample quality, wherein the expression levels of the general and prostate cell specific reference genes are used to provide at least one quality control filter, wherein only samples which pass the at least one quality control filter are included in analysis.

8. A nucleic acid that hybridizes under stringent conditions to one of the nucleic acids according to claim 1, 2 or 3.

9. The nucleic acid according to claim 8, wherein the nucleic acid is about 10 to 100 nucleotides in length.

10. The nucleic acid according to claim 8 or 9, wherein the nucleic acid comprises a detectable label.

**11.** Use of a nucleic acid according to any of claims 8 to 10 for the diagnosis of prostate cancer.

**12.** A nucleic acid with one of the sequences of SEQ ID NO: 1-131, or the reverse complement thereof or any part thereof, or a nucleic acid that shares preferably at least 85%, 90%, 95% or 99% sequence identity with one of the sequences of SEQ: 1-131, or the reverse complement thereof or any part thereof, preferably the nucleic acid is selected from the group comprising SEQ ID No: 136-174.

**13.** Use of a nucleic acid according to claim 12 for the diagnosis of prostate cancer.

**14.** A kit for the diagnosis of prostate cancer comprising a nucleic acid according to any one of claims 8 to 10 or a nucleic acid according to claim 12 and reagents for nucleic acid amplification and/or quantification and/or detection.

**15.** A method for the treatment and diagnosis of prostate cancer comprising the steps of

a) analysing the expression level of the nucleic acid according to any one of claims 1, 2 or 3 in a sample from a patient,
b) wherein, if the expression level of said nucleic acid is above a threshold value, the sample is designated as prostate cancer positive; and
c) administering to the patient one or more Prostate Cancer Therapeutic Agents.

Figure 1

## ProstaCheck
## cohorts

Discovery – RNA Seq
RP-cryopreserved tissue (n = 48)
BPH = 8, Tu = 40

⬇

Confirmation – Microarrays
RP-cryopreserved tissue (n = 203)
BPH = 39, Tu = 164

⬇

Confirmation in Urine – RNA Seq
Urinary Sediment (n = 11)
BPH = 6, Tu = 5

⬇

Training 1 – RT qPCR
Urinary sediment (n = 66)
BPH = 21, Tu = 45

⬇

Training 2 – RT qPCR
Urinary sediment (n = 120)
BPH = 38, Tu = 82

⬇

Training 3 – RT qPCR
Urinary sediment (n = 65)
BPH = 24, Tu = 41

⬇

Test – RT qPCR (IVC)
Urinary sediment (n = 87)
BPH = 43, Tu = 44

**Figure 2**

**Figure 3**

**Urinary sediment
(RNA-seq)**

Normalised TPM (log-scaled)

# EUROPEAN SEARCH REPORT

**Application Number**

EP 22 19 3923

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/082418 A1 (FRAUNHOFER GES FORSCHUNG [DE]) 11 June 2015 (2015-06-11) * the whole document * | 1,4-15 | INV. C12Q1/6886 |
| A | WO 2016/087430 A1 (FRAUNHOFER GES FORSCHUNG [DE]) 9 June 2016 (2016-06-09) * the whole document * | 1,4-15 | |
| A | WO 2015/057806 A1 (UNIV ILLINOIS [US]; UNIV LELAND STANFORD JUNIOR [US]) 23 April 2015 (2015-04-23) * the whole document * | 1,4-15 | |
| A | WO 2019/055648 A1 (TUFTS MEDICAL CT INC [US]) 21 March 2019 (2019-03-21) * the whole document * | 1,4-15 | |
| Y | WO 2020/169832 A1 (WIKSTRÖM PERNILLA [SE]; BERGH ANDERS [SE]; THYSELL ELIN [SE]) 27 August 2020 (2020-08-27) * the whole document * | 1,4-7 | TECHNICAL FIELDS SEARCHED (IPC) |
| Y | SHUKLA SUDHANSHU ET AL: "Identification and Validation of PCAT14 as Prognostic Biomarker in Prostate Cancer", NEOPLASIA, vol. 18, no. 8, 1 August 2016 (2016-08-01) , pages 489-499, XP093021808, US ISSN: 1476-5586, DOI: 10.1016/j.neo.2016.07.001 * the whole document * | 1,4-7 | C12Q |

-/--

The present search report has been drawn up for all claims

| Place of search | Date of completion of the search | Examiner |
|---|---|---|
| The Hague | 8 February 2023 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 22 19 3923

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SHUKLA KAMLA KANT ET AL: "Recent scenario of long non-coding RNAs as a diagnostic and prognostic biomarkers of prostate cancer", UROLOGIC ONCOLOGY: SEMINARS AND ORIGINAL INVESTIGATIONS, ELSEVIER, AMSTERDAM, NL, vol. 38, no. 12, 1 July 2020 (2020-07-01), pages 918-928, XP086388837, ISSN: 1078-1439, DOI: 10.1016/J.UROLONC.2020.06.003 [retrieved on 2020-07-01] * the whole document * | 1,4-7 | |
| | ----- | | |

|  |  |
|---|---|
| | TECHNICAL FIELDS SEARCHED (IPC) |

The present search report has been drawn up for all claims

| Place of search | Date of completion of the search | Examiner |
|---|---|---|
| The Hague | 8 February 2023 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

    **see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

    **1(completely); 4-15(partially)**

☐ The present supplementary European search report has been drawn up for those parts of the European patent application which relate to the invention first mentioned in the claims (Rule 164 (1) EPC).

The Search Division considers that the present European patent application does not comply with the requirements of unity of invention and relates to several inventions or groups of inventions, namely:

1. claims: 1(completely); 4-15(partially)

   INVENTION 1:
   A method for the ex-vivo diagnosis of prostate-cancer comprising the steps of (a.) providing a sample from a patient suspected of having prostate cancer, (b.) analyzing the expression level of at least the nucleic acids according to SEQ. ID. NO:2 and SEQ. ID. NO:20 in the sample, wherein, if the expression level of said nucleic acid is above a threshold value, the sample is designated as prostate cancer positive, the sequences as such according to claim 12, (SEQ. ID. NO:2 and SEQ. ID. NO:20), the use of said sequences for the diagnosis of prostate cancer as described in claims 11 and 13 (SEQ. ID. NO:2 and SEQ. ID. NO:20), a kit comprising said sequences as detailed in claim 14 (SEQ. ID. NO:2 and SEQ. ID. NO:20), and a method for the treatment and diagnosis of prostate cancer according to claim 15 and encompassing said sequences (SEQ. ID. NO:2 and SEQ. ID. NO:20).
   ---

2. claims: 2(completely); 4-15(partially)

   INVENTION NUMBER 2:
   A method for the ex-vivo diagnosis of prostate cancer comprising the steps of a) providing a sample from a patient suspected of having prostate cancer, b) analysing the expression level of at least the nucleic acids according to SEQ ID NO: 2, SEQ ID NO: 19-20 and SEQ ID NO: 35-37 in the sample, wherein, if the expression level of said nucleic acids is above a threshold value, the sample is designated as prostate cancer positive, the sequences as such according to claim 12, (SEQ ID NO: 2, SEQ ID NO: 19-20 and SEQ ID NO: 35-37 ), the use of said sequences for the diagnosis of prostate cancer as described in claims 11 and 13 (SEQ ID NO: 2, SEQ ID NO: 19-20 and SEQ ID NO: 35-37 ), a kit comprising said sequences as detailed in claim 14 (SEQ ID NO: 2, SEQ ID NO: 19-20 and SEQ ID NO: 35-37 ), and a method for the treatment and diagnosis of prostate cancer according to claim 15 and encompassing said sequences (SEQ ID NO: 2, SEQ ID NO: 19-20 and SEQ ID NO: 35-37 ).
   ---

3. claims: 3(completely); 4-15(partially)

   INVENTION NUMBER 3:
   A method for the ex-vivo diagnosis of prostate cancer comprising the steps of a) providing a sample from a patient suspected of having prostate cancer, b) analysing the expression level of at least the nucleic acids according to SEQ ID NO: 1-3, SEQ ID NO: 20-34 and SEQ ID NO: 38-50 in the

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

```
sample, wherein, if the expression level of said nucleic
acids is above a threshold value, the sample is designated
as prostate cancer positive, the sequences as such according
to claim 12, (SEQ ID NO: 1-3, SEQ ID NO: 20-34 and SEQ ID
NO: 38-50 ), the use of said sequences for the diagnosis of
prostate cancer as described in claims 11 and 13 (SEQ ID NO:
1-3, SEQ ID NO: 20-34 and SEQ ID NO: 38-50 ), a kit
comprising said sequences as detailed in claim 14 (SEQ ID
NO: 1-3, SEQ ID NO: 20-34 and SEQ ID NO: 38-50 ), and a
method for the treatment and diagnosis of prostate cancer
according to claim 15 and encompassing said sequences (SEQ
ID NO: 1-3, SEQ ID NO: 20-34 and SEQ ID NO: 38-50 ).
                            ---
```

**page 2 of 2**

## ANNEX TO THE EUROPEAN SEARCH REPORT
## ON EUROPEAN PATENT APPLICATION NO.

EP 22 19 3923

08-02-2023

| Patent document cited in search report | | Publication date | Patent family member(s) | | Publication date |
|---|---|---|---|---|---|
| WO 2015082418 | A1 | 11-06-2015 | EP | 2878678 A1 | 03-06-2015 |
| | | | EP | 3077530 A1 | 12-10-2016 |
| | | | EP | 3077531 A1 | 12-10-2016 |
| | | | EP | 3077535 A1 | 12-10-2016 |
| | | | EP | 3077536 A1 | 12-10-2016 |
| | | | EP | 3077537 A1 | 12-10-2016 |
| | | | ES | 2726667 T3 | 08-10-2019 |
| | | | ES | 2732371 T3 | 22-11-2019 |
| | | | ES | 2784246 T3 | 23-09-2020 |
| | | | US | 2016298199 A1 | 13-10-2016 |
| | | | US | 2016304965 A1 | 20-10-2016 |
| | | | US | 2016304966 A1 | 20-10-2016 |
| | | | US | 2016304967 A1 | 20-10-2016 |
| | | | US | 2016312291 A1 | 27-10-2016 |
| | | | WO | 2015082414 A1 | 11-06-2015 |
| | | | WO | 2015082416 A1 | 11-06-2015 |
| | | | WO | 2015082417 A1 | 11-06-2015 |
| | | | WO | 2015082418 A1 | 11-06-2015 |
| | | | WO | 2015082420 A1 | 11-06-2015 |
| WO 2016087430 | A1 | 09-06-2016 | CA | 2969154 A1 | 09-06-2016 |
| | | | CN | 107223162 A | 29-09-2017 |
| | | | EP | 3227460 A1 | 11-10-2017 |
| | | | JP | 2018505656 A | 01-03-2018 |
| | | | KR | 20170116009 A | 18-10-2017 |
| | | | US | 2017362662 A1 | 21-12-2017 |
| | | | WO | 2016087430 A1 | 09-06-2016 |
| WO 2015057806 | A1 | 23-04-2015 | EP | 3058101 A1 | 24-08-2016 |
| | | | US | 2016237505 A1 | 18-08-2016 |
| | | | WO | 2015057806 A1 | 23-04-2015 |
| WO 2019055648 | A1 | 21-03-2019 | CN | 111417855 A | 14-07-2020 |
| | | | US | 2021122833 A1 | 29-04-2021 |
| | | | WO | 2019055648 A1 | 21-03-2019 |
| WO 2020169832 | A1 | 27-08-2020 | EP | 3927850 A1 | 29-12-2021 |
| | | | EP | 3927851 A1 | 29-12-2021 |
| | | | US | 2022170105 A1 | 02-06-2022 |
| | | | US | 2022213552 A1 | 07-07-2022 |
| | | | WO | 2020169831 A1 | 27-08-2020 |
| | | | WO | 2020169832 A1 | 27-08-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015082418 A1

### Non-patent literature cited in the description

- **AUBIN SMJ et al.** *J Urol.,* 2010, vol. 184, 1947-52
- **YOAV BENJAMINI ; YOSEF HOCHBERG.** Controlling false discovery rate: a practical and powerful approach to multiple testing. *Journal of the Royal Statistical Society,* 1995
- **CUCCHIARA, V. ; COOPERBERG, M. R. ; DALL'ERA, M. ; LIN, D. W. ; MONTORSI, F. ; SCHALKEN, J. A. ; EVANS, C. P.** Genomic Markers in Prostate Cancer Decision Making. *European urology,* 2017
- **DUFFY, MICHAEL J.** Biomarkers for prostate cancer: prostate-specific antigen and beyond. *Clinical Chemistry and Laboratory Medicine (CCLM),* 2020
- The Veteran's Administration Cooperative Urologic Research Group: histologic grading and clinical staging of prostatic carcinoma. **GLEASON, D. F. ; TANNENBAUM, M.** Urologic Pathology: The Prostate. Lea and Febiger, 1977, 171-198
- **GUESCINI M ; SISTI D ; ROCCHI MB ; STOCCHI L ; STOCCHI V.** A new real-time PCR method to overcome significant quantitative inaccuracy due to slight amplification inhibition. *BMC Bioinformatics,* 30 July 2008, vol. 9, 326
- **HAESE A et al.** *Eur Urol.,* 2008, vol. 54, 1081-8
- **IRSHAD S et al.** *Sci Transl Med,* 2013, vol. 5, 202-122

- **JANSEN FH et al.** *Eur Urol.,* 2009, vol. 55, 563-74
- **JANSEN FH ; VAN SCHAIK RH ; KURSTJENS J ; HORNINGER W ; KLOCKER H ; BEKTIC J et al.** Prostate-specific antigen (PSA) isoform p2PSA in combination with total PSA and free PSA improves diagnostic accuracy in prostate cancer detection. *Eur Urol,* 2010, vol. 57, 921-7
- **LENDINEZ-CANO G.** Prospective study of diagnostic accuracy in the detection of high-grade prostate cancer in biopsy-naive patients with clinical suspicion of prostate cancer who underwent the Select MDx test. *Prostate,* September 2021, vol. 81 (12), 857-865
- **MOYER VA et al.** *Ann Intern Med.,* 2012, vol. 157, 120-134
- **SALAGIERSKI M et al.** *J Urol,* 2012, vol. 187, 795-801
- **THOMPSON IM et al.** *J Am Med Assoc.,* 2005, vol. 294, 66-70
- **VAN GILS MP et al.** *Clin Cancer Res.,* 2007, vol. 13, 939-43
- **VICKERS AJ ; CRONIN AM ; AUS G ; PIHL CG ; BECKER C ; PETTERSSON K et al.** A panel of kallikrein markers can reduce unnecessary biopsy for prostate cancer: data from the European Randomized Study of Prostate Cancer Screening in Goteborg, Sweden. *BMC Med,* 2008, vol. 6, 19
- **TANNENBAUM, M.** Urologic Pathology: The Prostate. Lea and Febiger, 171-198